(19) Europäisches Patentamt
European Patent Office
Office européen des brevets

(11) Veröffentlichungsnummer : **0 638 869 B1**

# (12) EUROPÄISCHE PATENTSCHRIFT

(45) Veröffentlichungstag der Patentschrift :
**07.06.95 Patentblatt 95/23**

(51) Int. Cl.[6] : **G06F 17/00**

(21) Anmeldenummer : **93113022.3**

(22) Anmeldetag : **13.08.93**

## (54) Verfahren zur hochauflösenden Spektralanalyse bei mehrkanaligen Beobachtungen.

(43) Veröffentlichungstag der Anmeldung :
**15.02.95 Patentblatt 95/07**

(45) Bekanntmachung des Hinweises auf die Patenterteilung :
**07.06.95 Patentblatt 95/23**

(84) Benannte Vertragsstaaten :
**DE FR GB IT NL**

(56) Entgegenhaltungen :
**PROCEEDINGS OF THE 1983 INTERNATIONAL CONFERENCE ON SYSTEMS, MAN AND CYBERNETICS, IEEE PRESS NEW YORK US, Bd.1, 29. Dezember 1983, BOMBAY AND NEW DELHI, INDIA Seiten 408 - 413 S.Y.KUNG ET AL 'A REALIZATION APPROACH TO SPECTRAL LINE ESTIMATION'**
**PROCEEDINGS OF THE IEEE, Bd.80, Nr.2, Februar 1992, NEW YORK, US Seiten 283 - 309, XP000291184 BHASKAR D. RAO ET AL 'MODEL BASED PROCESSING OF SIGNALS: A STATE SPACE APPROACH'**
**PHYSIOLOGICAL RESEARCH, Bd.42, Nr.2, 1993 Seiten 73 - 76 Z.DRSKA ET AL 'CARDIAC MICROPOTENTIALS REACHED FROM ONE SYSTOLE AS NONDIPOLAR RESIDUE BY SINGULAR VALUE DECOMPOSITION'**
**PROCEEDINGS OF THE 1985 EUROPEAN CONFERENCE ON CIRCUIT THEORY AND DESIGN, NORTH HOLLAND NL, 2. September 1985, PRAGUE, CZECHOSLOVAKIA Seiten 458 - 461 J.VANDEWALLE ET AL 'FILTERING OF VECTOR SIGNALS BASED ON THE SINGULAR VALUE DECOMPOSITION'**
**PROCEEDINGS OF THE SIXTH INTERNATIONAL CONFERENCE ON ANALYSIS AND OPTIMIZATION OF SYSTEMS, SPRINGER VERLAG, GERMANY, Bd.2, 19. Juni 1984, NICE, FRANCE Seiten 434 - 448 J.VANDERSCHOOT ET AL 'EXTRACTION OF WEAK BIOELECTRICAL SIGNALS BY MEANS OF SINGULAR VALUE DECOMPOSITION'**

(56) Entgegenhaltungen :
**JOURNAL A, Bd.32, Nr.2, Juni 1991, ANTWERPEN BE Seiten 11 - 16, XP000237329 D.CALLAERTS 'SINGULAR VALUE DECOMPOSITION IN DIGITAL SIGNAL PROCESSING'**

(73) Patentinhaber : **SIEMENS AKTIENGESELLSCHAFT**
**Wittelsbacherplatz 2**
**D-80333 München (DE)**

(72) Erfinder : **Haardt, Martin, Dipl.-Ing.**
**Avenariusstrasse 18**
**D-81243 München (DE)**
Erfinder : **Strobach, Peter, Dr.-Ing.**
**Josef-Maria-Lutz-Anger 32**
**D-81737 München (DE)**

**EP 0 638 869 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur hochauflösenden Spektralanalyse bei mehrkanaligen Beobachtungen. Verfahren zur Spektralanalyse werden benötigt, um - möglicherweise gedämpfte - harmonische Anteile in Signalen verschiedenster Herkunft aufzufinden und zu identifizieren. Die Erfindung betrifft ferner die Verwendung eines solchen Verfahrens zur Detektion von ventrikulären Spätpotentialen in mehrkanaligen medizintechnischen Meßverfahren, bei denen vom Herzen stammende Signale ausgewertet werden. Wichtige Beispiele hierfür sind die Magnetokardiographie (MKG) oder die Elektrokardiographie (EKG).

Bekannte Verfahren zur Spektralanalyse und ihre Verwendung zur Identifikation von Spätpotentialen wurden in den Veröffentlichungen von D.E. Balderson, et. al., "The detection, significance and effect of drugs upon ventricular late potentials", Automedia, vol. 13, pp. 67-96, 1991 und E.J. Berbari, R. Lazzara, "An Introduction to High-Resolution ECG Recordings of Cardiac Late Potentials", Arch. Intern. med., vol. 148, pp. 1859-1863, August 1988, beschrieben. Ferner geben die Aufsatze von S.M. Kay, S.L. Marple, "Spectrum Analysis - A Modern Perspective", Proceedings of the IEEE, Vol. 69, No. 11, November 1981, B.D. Rao, K.S. Arun, "Model based Processing of Signals: A State Space Approach", Proc. IEEE, vol. 80, pp. 283-309, Feb. 1992 sowie S. Haykin, Editor, "Nonlinear Methods of Spectral Analysis", Topics in Applied Physics, vol. 34, Springer-Verlag, 1979, eine gute Übersicht über die bekannten Verfahren zur Spektralanalyse.

Diese bekannten Verfahren haben jeweils bestimmte Stärken und Schwächen. Mit keinem dieser bekannten Verfahren ist es jedoch möglich, ventrikuläre Spätpotentiale in Einzelschlägen unter Verwendung mehrkanaliger Meßdaten nachzuweisen.

Der Erfindung liegt die Aufgabe zugrunde, ein Verfahren zur hochauflösenden Spektralanalyse bei mehrkanaligen Beobachtungen anzugeben, mit dem ventrikuläre Spätpotentiale in Einzelschlägen nachgewiesen werden können.

Diese Aufgabe wird erfindungsgemäß durch ein Spektralanalyseverfahren zur hochauflösenden Spektralanalyse bei mehrkanaligen Beobachtungen mit Merkmalen nach Anspruch 1, 2, 3 oder 4 gelöst. Mit dem Verfahren ist der Nachweis ventrikulärer Spätpotentiale in Einzelschlägen möglich. Das Verfahren bietet ferner die Möglichkeit zur räumlichen Lokalisierung pathologischer Veränderungen, die in ursächlichem Zusammenhang mit der Entstehung dieser Spätpotentiale stehen. Dieses Verfahren hat den bemerkenswerten Vorteil, völlig unabhängig von der Geometrie des verwendeten Sensorfeldes zu sein.

Vorteilhafte Weiterbildungen der Erfindung ergeben sich aus den Unteransprüchen.

Figur 1 zeigt in schematischer Darstellung den zeitlichen Verlauf eines Herzschlagsignals, speziell den sogenannten QRS-Komplex und die ihm nachfolgende ST-Strecke.

Figur 2 zeigt in schematischer Darstellung die Kompensation eines Gleichanteils und eines linearen Trends im Meßsignal.

Die Figuren 3 und 4 zeigen mit dem erfindungsgemäßen Verfahren ermittelte Frequenzhistogramme von Patienten mit Spätpotentialen.

Die Figuren 5 und 6 zeigen mit dem erfindungsgemäßen Verfahren ermittelte Frequenzhistogramme von Patienten ohne Spätpotentiale.

Dabei ist jeweils die Zahl der gefundenen Freuquenzen (Ordinate) über der Freuquenz in Hertz (Abszisse) aufgetragen.

Im folgenden wird die Erfindung anhand bevorzugter Ausführungsbeispiele und mit Hilfe der Figuren näher beschrieben.

Bei der Erfindung handelt es sich um ein neues, hochauflösendes Verfahren zur Schatzung mehrerer überlagerter, möglicherweise gedämpfter Exponentialschwingungen aus mehrkanaligen, rauschbehafteten Beobachtungen. Die erfindungsgemäße Lösung basiert auf einer Zustandsdarstellung der Meßsignale, wodurch das nichtlineare Schatzproblem linear parametrisiert wird. Bemerkenswerterweise ist das Verfahren unabhängig von der räumlichen Anordnung der zur Meßdatenerhebung verwendeten Sensoren und kann daher bei einer beliebigen, vorgegebenen aber möglicherweise unbekannten Geometrie des Sensorfeldes angewendet werden.

Das erfindungsgemäße Verfahren ist in seiner Anwendbarkeit nicht auf die Detektion von ventrikulären Spätpotentialen beschränkt, sondern kann in ähnlicher Weise auf Probleme der Radar- oder Sonartechnik, der Astronomie, der Vermessung von Mobilfunkkanälen oder auf andere Probleme der seismischen oder medizinischen Signalverarbeitung angewendet werden.

Um das spektrale Schatzproblem mathematisch zu beschreiben, wird angenommen, daß M Sensoren die Summe aus p, möglicherweise gedämpften, Exponentialschwingungen empfangen. Nennt man das am i-ten Sensor ($\mathbf{1 \leqq \mathit{i} \leqq \mathit{M}}$, mit $\mathbf{M} > 1$) zum Zeitpunkt n (mit $\mathbf{0 \leqq \mathit{n} \leqq \mathit{N}-1}$) beobachtete Signal $\boldsymbol{y_i(n)}$, können die rauschfreien Beobachtungen wie folgt beschrieben werden:

$$y_i(n) = \sum_{\nu=1}^{p} c_{\nu,i} \cdot \lambda_\nu^n = \sum_{\nu=1}^{p} c_{\nu,i} \cdot e^{(\gamma_\nu + j\omega_\nu)n}$$

Hierbei bezeichnet $\mathbf{c}_{\nu,i}$ die unbekannte komplexwertige Amplitude der ν-ten Exponentialschwingung im i-ten Kanal, und $\gamma_\nu$ bzw. $\omega_\nu$ können als Dämpfung bzw. Frequenz der ν-ten Exponentialschwingung interpretiert werden. Offensichtlich können auch reelle Signale behandelt werden, da sich reelle harmonische Schwingungen als Summe zweier (komplexwertiger) Exponentialschwingungen darstellen lassen.

Im folgenden werden die Beobachtungen an den M Sensoren zu einem Datenvektor

$$\mathbf{y}(n) = (y_1(n),\cdots,y_M(n))^T$$

zusammengefaßt. Hierbei wird folgende Notation verwendet: Spaltenvektoren bzw. Matrizen werden durch fettgedruckte Klein- bzw. Großbuchstaben gekennzeichnet. Transponierte, bzw. transjugierte, Matrizen und Vektoren erhalten den Zusatz T bzw. H.

Ventrikuläre Spätpotentiale sind hochfrequente Signale mit kleinen Amplituden im terminalen Anteil des QRS-Komplexes und in der ST-Strecke des Herzschlags (s. Fig. 1). Diese pathologischen Signalanteile entsprechen einer regional verzögerten, in Abständen erfolgenden Erregungsausbreitungen. Sie werden oft bei Patienten mit ventrikulären Tachykardien erfaßt. Ihre Bedeutung für die Diagnostik liegt insbesondere in der Risikobeurteilung von Patienten mit ventrikulären Arrhythmien und in der Therapie bzw. Therapiekontrolle. Das erfindungsgemäße Verfahren liefert eine geeignete Methode zum Nachweis von Spätpotentialen aus mehrkanaligen Meßreihen.

Spätpotentiale lassen sich sowohl in Elektrokardiogrammen (EKG) als auch in Magnetokardiogrammen (MKG) nachweisen, wobei bei beiden Meßarten prinzipiell die gleichen Verfahren angewendet werden können. Im Unterschied zum EKG stehen bei der biomagnetischen (MKG) Messungen jedoch sehr viel mehr Kanäle, typischerweise bis zu 37 gleichzeitig aufgezeichnete Meßkanäle, zur Verfügung. Da die Meßdaten in diesen Kanälen in hohem Maße korreliert sind, bieten sie die Voraussetzung für einen wesentlich zuverlässigeren Nachweis der Spätpotentiale als bei einkanaligen Meßverfahren.

Bisher werden Spätpotentiale mit Hilfe von Signalmittelungstechniken, bzw. einer auf der schnellen Fourier Transformation basierenden Frequenzanalyse identifiziert. Diese Verfahren sind jedoch nicht geeignet ventrikuläre Spätpotentiale in Einzelschlägen nachzuweisen.

Das erfindungsgemäße Verfahren kann in zwei Varianten ausgeführt werden, welche von zwei unterschiedlichen Zustandsdarstellungen der Signale ausgehen: Eine erste Variante des erfindungsgemäßen Verfahrens geht von der sogenannten erweiterten Zustandsdarstellung der Signale aus. Hierzu werden die Meßdaten in Form einer Matrix

$$\mathbf{Y}_E = \begin{pmatrix} \mathbf{y}^T(0) & \mathbf{y}^T(1) & \cdots & \mathbf{y}^T(N-L) \\ \mathbf{y}^T(1) & \mathbf{y}^T(2) & \cdots & \mathbf{y}^T(N-L+1) \\ \vdots & \vdots & & \vdots \\ \mathbf{y}^T(L-1) & \mathbf{y}^T(L) & \cdots & \mathbf{y}^T(N-1) \end{pmatrix}$$

dargestellt, wogegen die andere Variante des erfindungsgemäßen Verfahrens von der sogenannten traditionellen Zustandsdarstellung, gegeben durch die Matrix

$$\mathbf{Y}_T = \begin{pmatrix} \mathbf{y}(0) & \mathbf{y}(1) & \cdots & \mathbf{y}(N-L) \\ \mathbf{y}(1) & \mathbf{y}(2) & \cdots & \mathbf{y}(N-L+1) \\ \vdots & \vdots & & \vdots \\ \mathbf{y}(L-1) & \mathbf{y}(L) & \cdots & \mathbf{y}(N-1) \end{pmatrix}$$

ausgeht. Beide Darstellungen sind mathematisch äquivalent haben aber formal unterschiedliche Eigenschaften. In Abhängigkeit von den Werten der Parameter N, M und L wählt der Anwender des erfindungsgemäßen Verfahrens aus diesen beiden grundsätzlich gleichwertigen Alternativen diejenige Darstellung, die auf eine möglichst quadratische Matrix, d.h. eine Matrix mit möglichst kleiner Differenz zwischen Zeilen- und Spaltenzahl, fuhrt. Eine solche Wahl fuhrt nämlich zu optimalen numerischen Eigenschaften bei der Ausführung des erfindungsgemäßen Verfahrens.

Im folgenden werden die einzelnen Verfahrensschritte zur Durchführung des erfindungsgemäßen Verfahrens unter Verwendung der oben eingeführten Notation ausführlich dargestellt.

Eine erste Variante des erfindungsgemäßen Verfahrens zur hochauflösenden Spektralanalyse ist dadurch gekennzeichnet, daß

a) M zeitliche Folgen $y_i(n)$ von Meßdaten der Sensorkanäle $1 \leqq i \leqq M$ zu den Zeitpunkten $0 \leqq n \leqq N\text{-}1$ beobachtet und zu einer Matrix $\mathbf{Y}_E$ der Form

$$\mathbf{Y}_E = \begin{pmatrix} \mathbf{y}^T(0) & \mathbf{y}^T(1) & \cdots & \mathbf{y}^T(N-L) \\ \mathbf{y}^T(1) & \mathbf{y}^T(2) & \cdots & \mathbf{y}^T(N-L+1) \\ \vdots & \vdots & & \vdots \\ \mathbf{y}^T(L-1) & \mathbf{y}^T(L) & \cdots & \mathbf{y}^T(N-1) \end{pmatrix}$$

mit $\mathbf{y}(n) = (y_1(n),...,y_M(n))^T$ zusammengefaßt werden,

b) eine Singulärwertzerlegung $\mathbf{Y}_E = \mathbf{U} \cdot \Sigma \cdot \mathbf{V}^H$ der Matrix $\mathbf{Y}_E$ durchgeführt wird, und die Singulärwerte einer Schwellwertoperation unterzogen werden, wodurch alle Singulärwerte von $\mathbf{Y}_E$, die dem Betrage nach kleiner sind als der Schwellwert, durch Null ersetzt werden und wonach alle p dominanten, d.h. nicht durch Null ersetzten, Singulärwerte von $\mathbf{Y}_E$ in der Blockmatrix $\Sigma_1$ der Zerlegung

$$\mathbf{Y}_E = (\mathbf{U}_1 \quad \mathbf{U}_2) \cdot \begin{pmatrix} \mathbf{\Sigma}_1 & \mathbf{0} \\ \mathbf{0} & \mathbf{0} \end{pmatrix} \cdot \begin{pmatrix} \mathbf{V}_1^H \\ \mathbf{V}_2^H \end{pmatrix}$$

enthalten sind,

c) diejenigen Matrizen $\Theta_1$ und $\Theta_2$ bestimmt werden, für die

$$\mathbf{U}_1 \cdot \mathbf{\Sigma}_1^{1/2} = \begin{pmatrix} \Theta_1 \\ \theta_2 \end{pmatrix} = \begin{pmatrix} \theta_1 \\ \Theta_2 \end{pmatrix}$$

gilt, wobei $\theta_1$ und $\theta_2$ jeweils die erste bzw. die letzte Blockmatrix bezeichnen,

d) eine Matrix **F** bestimmt wird, die eine optimale Lösung des im allgemeinen überbestimmten Gleichungssystems $\Theta_1 \cdot \mathbf{F} = \Theta_2$ ist, wobei die Imaginärteile der natürlichen Logarithmen der Eigenwerte von **F** die gesuchten spektralen Frequenzen und die Realteile der natürlichen Logarithmen der Eigenwerte von **F** mit diesen Frequenzen assoziierte Dämpfungskonstanten sind.

Somit ist diese erste Variante des erfindungsgemäßen Verfahrens ein Verfahren zur hochauflösenden Spektralanalyse, bei dem M zeitliche Folgen $y_i(n)$ von Meßdaten der Sensorkanäle $1 \leqq i \leqq M$ zu den Zeitpunkten $0 \leqq n \leqq N\text{-}1$ beobachtet werden, dadurch gekennzeichnet, daß spektrale Frequenzen und mit diesen Frequenzen assoziierte Dämpfungskonstanten als Imaginär- bzw. Realteile der natürlichen Logarithmen der Eigenwerte einer Matrix **F** bestimmt werden, die als optimale Lösung eines im allgemeinen überbestimmten Gleichungssystems $\Theta_1 \cdot \mathbf{F} = \Theta_2$ definiert ist, wobei die Matrizen $\Theta_1$ und $\Theta_2$ definiert sind durch die Beziehung

$$\mathbf{U}_1 \cdot \mathbf{\Sigma}_1^{1/2} = \begin{pmatrix} \Theta_1 \\ \theta_2 \end{pmatrix} = \begin{pmatrix} \theta_1 \\ \Theta_2 \end{pmatrix},$$

in der $\theta_1$ und $\theta_2$ jeweils die erste bzw. die letzte Blockmatrix bezeichnen, und in der die Matrizen $\Sigma_1$ und $\mathbf{U}_1$ definiert sind durch eine approximative Singulärwertzerlegung

$$\mathbf{Y}_E = (\mathbf{U}_1 \quad \mathbf{U}_2) \cdot \begin{pmatrix} \mathbf{\Sigma}_1 & \mathbf{0} \\ \mathbf{0} & \mathbf{0} \end{pmatrix} \cdot \begin{pmatrix} \mathbf{V}_1^H \\ \mathbf{V}_2^H \end{pmatrix}$$

einer Matrix

$$\mathbf{Y}_E = \begin{pmatrix} \mathbf{y}^T(0) & \mathbf{y}^T(1) & \cdots & \mathbf{y}^T(N-L) \\ \mathbf{y}^T(1) & \mathbf{y}^T(2) & \cdots & \mathbf{y}^T(N-L+1) \\ \vdots & \vdots & & \vdots \\ \mathbf{y}^T(L-1) & \mathbf{y}^T(L) & \cdots & \mathbf{y}^T(N-1) \end{pmatrix},$$

wobei $\mathbf{y}(n) = (y_1(n),...,y_M(n))^T$, und wobei die approximative Singulärwertzerlegung aus einer ursprünglichen Singulärwertzerlegung $\mathbf{Y}_E = \mathbf{U}\cdot\Sigma\cdot\mathbf{V}^H$ dadurch ermittelt wird, daß die Singulärwerte einer Schwellwertoperation unterzogen werden, wodurch alle Singulärwerte von $\mathbf{Y}_E$, die dem Betrage nach kleiner sind als der Schwellwert, durch Null ersetzt werden und wonach alle p dominanten, d.h. nicht durch Null ersetzten, Singulärwerte von $\mathbf{Y}_E$ in der Blockmatrix $\Sigma_1$ der approximativen Zerlegung enthalten sind.

Diese erste Variante des erfindungsgemäßen Verfahrens basiert auf der sogenannten erweiterten Zustandsdarstellung. Diese Variante ist einer Erweiterung zugänglich, welche noch dargestellt wird, und durch welche sich die Genauigkeit der Spektralanalyse bei Abwesenheit von Dämpfungserscheinungen (verschwindende Dämpfungskonstanten) weiter verbessern läßt.

Eine grundsätzlich gleichwertige, formal andersartige, zweite Variante des erfindungsgemäßen Verfahrens zur hochauflösenden Spektralanalyse ist dadurch gekennzeichnet, daß

a) M zeitliche Folgen $y_i(n)$ von Meßdaten der Sensorkanäle $1 \leqq i \leqq M$ zu den Zeitpunkten $0 \leqq n \leqq N\text{-}1$ beobachtet und zu einer Matrix $\mathbf{Y}_T$ der Form

$$\mathbf{Y}_T = \begin{pmatrix} \mathbf{y}(0) & \mathbf{y}(1) & \cdots & \mathbf{y}(N-L) \\ \mathbf{y}(1) & \mathbf{y}(2) & \cdots & \mathbf{y}(N-L+1) \\ \vdots & \vdots & & \vdots \\ \mathbf{y}(L-1) & \mathbf{y}(L) & \cdots & \mathbf{y}(N-1) \end{pmatrix}$$

mit $\mathbf{y}(n) = (y_1(n),...,y_M(n))^T$ zusammengefaßt werden,

b) eine Singulärwertzerlegung $\mathbf{Y}_T = \mathbf{U}\cdot\Sigma\cdot\mathbf{V}^H$ der Matrix $\mathbf{Y}_T$ durchgeführt wird, und die Singulärwerte einer Schwellwertoperation unterzogen werden, wodurch alle Singulärwerte von $\mathbf{Y}_T$, die dem Betrage nach kleiner sind als der Schwellwert, durch Null ersetzt werden und wonach alle p dominanten, d.h. nicht durch Null ersetzten, Singulärwerte von $\mathbf{Y}_T$ in der Blockmatrix $\Sigma_1$ der Zerlegung

$$\mathbf{Y}_T = (\mathbf{U}_1 \quad \mathbf{U}_2) \cdot \begin{pmatrix} \mathbf{\Sigma}_1 & \mathbf{0} \\ \mathbf{0} & \mathbf{0} \end{pmatrix} \cdot \begin{pmatrix} \mathbf{V}_1^H \\ \mathbf{V}_2^H \end{pmatrix}$$

enthalten sind,

c) diejenigen Matrizen $\Theta_1$ und $\Theta_2$ bestimmt werden, für die

$$\mathbf{U}_1 \cdot \mathbf{\Sigma}_1^{1/2} = \begin{pmatrix} \Theta_1 \\ \theta_2 \end{pmatrix} = \begin{pmatrix} \theta_1 \\ \Theta_2 \end{pmatrix}$$

gilt, wobei $\theta_1$ und $\theta_2$ jeweils die erste bzw. die letzte Blockmatrix bezeichnen,

d) eine Matrix **F** bestimmt wird, die eine optimale Lösung des im allgemeinen überbestimmten Gleichungs-

systems $\Theta_1 \cdot \mathbf{F} = \Theta_2$ ist, wobei die Imaginärteile der natürlichen Logarithmen der Eigenwerte von **F** die gesuchten spektralen Frequenzen und die Realteile der natürlichen Logarithmen der Eigenwerte von **F** mit diesen Frequenzen assoziierte Dämpfungskonstanten sind.

Diese zweite Variante des erfindungsgemäßen Verfahrens läßt sich auch beschreiben als ein Verfahren zur hochauflösenden Spektralanalyse, bei dem M zeitliche Folgen $y_i(n)$ von Meßdaten der Sensorkanäle $1 \leqq i \leqq M$ zu den Zeitpunkten $0 \leqq n \leqq N-1$ beobachtet werden, dadurch gekennzeichnet, daß spektrale Frequenzen und mit diesen Frequenzen assoziierte Dämpfungskonstanten als Imaginär- bzw. Realteile der natürlichen Logarithmen der Eigenwerte einer Matrix **F** bestimmt werden, die als optimale Lösung eines im allgemeinen überbestimmten Gleichungssystems $\Theta_1 \cdot \mathbf{F} = \Theta_2$ definiert ist, wobei die Matrizen $\Theta_1$ und $\Theta_2$ definiert sind durch die Beziehung

$$\mathbf{U}_1 \cdot \Sigma_1^{1/2} = \begin{pmatrix} \Theta_1 \\ \theta_2 \end{pmatrix} = \begin{pmatrix} \theta_1 \\ \Theta_2 \end{pmatrix},$$

in der $\theta_1$ und $\theta_2$ jeweils die erste bzw. die letzte Blockmatrix bezeichnen, und in der die Matrizen $\Sigma_1$ und $\mathbf{U}_1$ definiert sind durch eine approximative Singulärwertzerlegung

$$\mathbf{Y}_T = \begin{pmatrix} \mathbf{U}_1 & \mathbf{U}_2 \end{pmatrix} \cdot \begin{pmatrix} \Sigma_1 & \mathbf{0} \\ \mathbf{0} & \mathbf{0} \end{pmatrix} \cdot \begin{pmatrix} \mathbf{V}_1^H \\ \mathbf{V}_2^H \end{pmatrix}$$

einer Matrix

$$\mathbf{Y}_T = \begin{pmatrix} \mathbf{y}(0) & \mathbf{y}(1) & \cdots & \mathbf{y}(N-L) \\ \mathbf{y}(1) & \mathbf{y}(2) & \cdots & \mathbf{y}(N-L+1) \\ \vdots & \vdots & & \vdots \\ \mathbf{y}(L-1) & \mathbf{y}(L) & \cdots & \mathbf{y}(N-1) \end{pmatrix},$$

wobei $\mathbf{y}(n) = (y_1(n),...,y_M(n))^T$, und wobei die approximative Singulärwertzerlegung aus einer ursprünglichen Singulärwertzerlegung $\mathbf{Y}_T = \mathbf{U} \cdot \Sigma \cdot \mathbf{V}^H$ dadurch ermittelt wird, daß die Singulärwerte einer Schwellwertoperation unterzogen werden, wodurch alle Singulärwerte von $\mathbf{Y}_T$, die dem Betrage nach kleiner sind als der Schwellwert, durch Null ersetzt werden und wonach alle p dominanten, d.h. nicht durch Null ersetzten, Singulärwerte von $\mathbf{Y}_T$ in der Blockmatrix $\Sigma_1$ der approximativen Zerlegung enthalten sind.

Diese zweite Variante des erfindungsgemäßen Verfahrens basiert auf der sogenannten traditionellen Zustandsdarstellung.

Hiermit sind zwei Varianten des erfindungsgemäßen Verfahrens zur Bestimmung von spektralen Frequenzen und mit diesen assoziierten Dämpfungskonmstanten beschrieben worden. Die Figuren 3, 4, 5 und 6 zeigen in schematischer Weise verschiedene Frequenzhistogramme, die mit dem erfindungsgemäßen Verfahren ermittelt wurden. Dabei zeigen die Figuren 3 und 4 Frequenzhistogramme von Patienten mit Spätpotentialen. Diese sind an der Häufung hoher Frequenzanteile um ca. 200 Hertz leicht zu erkennen. In den Figuren 5 und 6, welche Frequenzhistogramme von Probanden ohne Spätpotentiale zeigen, fehlen diese Häufungen am oberen Ende des Spektrums.

In manchen Fällen ist der Anwender aber nicht nur an den Frequenzen und Dämpfungskonstanten, sondern auch an den Amplituden und Phasen der spektralen Schwingungen interessiert. Diese lassen sich in dem Fachmann bekannter Weise aus komplexwertigen Amplituden dieser Schwingungen berechnen.

Eine bevorzugte Ausführungsform der Erfindung ist daher in einem Verfahren gemäß der oben beschriebenen ersten Variante zu sehen, die dadurch gekennzeichnet ist, daß die Matrix **C** der komplexwertigen Amplituden $c_{j,i}$ der mit den spektralen Frequenzen j = 1,...,p assoziierten Schwingungen im Sensorkanal i = 1,...,M durch die Ausführung der folgenden Schritte ermittelt wird:

a) ausgehend von der Ähnlichkeitstransformation $\mathbf{F} = \mathbf{S} \cdot \Lambda \cdot \mathbf{S}^{-1}$ der Matrix **F** und deren Diagonalform $\Lambda$ werden die Matrizen

$$\mathbf{U}_1 \cdot \Sigma_1^{1/2} \cdot \mathbf{S} \quad \text{und} \quad \mathbf{S}^{-1} \cdot \Sigma_1^{1/2} \cdot \mathbf{V}_1^H$$

aufgestellt und in Blockmatrizen

$$\mathbf{U}_1 \cdot \boldsymbol{\Sigma}_1^{1/2} \cdot \mathbf{S} = \begin{bmatrix} \mathbf{v}_1^T \\ \mathbf{v}_2^T \\ \mathbf{v}_3^T \\ \vdots \\ \mathbf{v}_L^T \end{bmatrix} \quad \text{sowie} \quad \mathbf{S}^{-1} \cdot \boldsymbol{\Sigma}_1^{1/2} \cdot \mathbf{V}_1^H = [\mathbf{W}_1 \quad \mathbf{W}_2 \quad \mathbf{W}_3 \quad \cdots \quad \mathbf{W}_{N-L+1}]$$

unterteilt;

b) aus diesen Darstellungen werden die Matrizen

$$\begin{bmatrix} \mathbf{v}_1^T \\ \mathbf{v}_2^T \cdot \Lambda^{-1} \\ \mathbf{v}_3^T \cdot \Lambda^{-2} \\ \vdots \\ \mathbf{v}_L^T \cdot \Lambda^{-(L-1)} \end{bmatrix}$$

sowie $[\mathbf{W}_1\ \Lambda^{-1} \cdot \mathbf{W}_2\ \Lambda^{-2} \cdot \mathbf{W}_3 \cdots \Lambda^{-(N-L)} \cdot \mathbf{W}_{N-L+1}]$ abgeleitet;

c) die Matrix **C** der komplexen Amplituden wird dann nach der Beziehung $\mathbf{C} = \mathbf{diag}\{h_1, h_2, ..., h_p\} \cdot \mathbf{X}_D$ ermittelt, wobei $\mathbf{h}^T = (h_1, h_2, ..., h_p)$ den Mittelwert der Zeilenvektoren von

$$\begin{bmatrix} \mathbf{v}_1^T \\ \mathbf{v}_2^T \cdot \Lambda^{-1} \\ \mathbf{v}_3^T \cdot \Lambda^{-2} \\ \vdots \\ \mathbf{v}_L^T \cdot \Lambda^{-(L-1)} \end{bmatrix}$$

und $\mathbf{X}_D$ den Mittelwert der Blockmatrizen in $[\mathbf{W}_1\ \Lambda^{-1} \cdot \mathbf{W}_2\ \Lambda^{-2} \cdot \mathbf{W}_3 \cdots \Lambda^{-(N-L)} \cdot \mathbf{W}_{N-L+1}]$ bezeichnet.

Dazu gleichwertig ist eine entsprechende Weiterbildung der oben beschriebenen zweiten Variante des erfindungsgemäßen Verfahrens, die dadurch gekennzeichnet ist, daß die Matrix **C** der komplexwertigen Amplituden $c_{j,i}$ der mit den spektralen Frequenzen j = 1,...,p assoziierten Schwingungen im Sensorkanal i = 1,...,M durch die Ausführung der folgenden Schritte ermittelt wird:

a) ausgehend von der Ähnlichkeitstransformation $\mathbf{F} = \mathbf{S} \cdot \Lambda \cdot \mathbf{S}^{-1}$ der Matrix **F** und deren Diagonalform $\Lambda$ werden die Matrizen

$$\mathbf{U}_1 \cdot \boldsymbol{\Sigma}_1^{1/2} \cdot \mathbf{S} \quad \text{und} \quad \mathbf{S}^{-1} \cdot \boldsymbol{\Sigma}_1^{1/2} \cdot \mathbf{V}_1^H$$

aufgestellt und in Blockmatrizen

$$\mathbf{U}_1 \cdot \boldsymbol{\Sigma}_1^{1/2} \cdot \mathbf{S} = \begin{bmatrix} \mathbf{V}_1^T \\ \mathbf{V}_2^T \\ \mathbf{V}_3^T \\ \vdots \\ \mathbf{V}_L^T \end{bmatrix} \quad \text{sowie} \quad \mathbf{S}^{-1} \cdot \boldsymbol{\Sigma}_1^{1/2} \cdot \mathbf{V}_1^H = \begin{bmatrix} \mathbf{w}_1 & \mathbf{w}_2 & \mathbf{w}_3 & \cdots & \mathbf{w}_{N-L+1} \end{bmatrix}$$

unterteilt;

b) aus diesen Darstellungen werden die Matrizen

$$\begin{bmatrix} \mathbf{V}_1^T \\ \mathbf{V}_2^T \cdot \Lambda^{-1} \\ \mathbf{V}_3^T \cdot \Lambda^{-2} \\ \vdots \\ \mathbf{V}_L^T \cdot \Lambda^{-(L-1)} \end{bmatrix}$$

sowie $[\mathbf{w}_1 \; \Lambda^{-1} \cdot \mathbf{w}_2 \; \Lambda^{-2} \cdot \mathbf{w}_3 \cdots \Lambda^{-(N-L)} \cdot \mathbf{w}_{N-L+1}]$ abgeleitet;

c) die Matrix **C** der komplexen Amplituden wird dann nach der Beziehung $\mathbf{C}^T = \mathbf{H}_D \cdot \mathbf{diag}\{x_1, x_2, \ldots, x_p\}$ ermittelt, wobei $\mathbf{x} = (x_1, x_2, \ldots, x_p)^T$ den Mittelwert der Spaltenvektoren von $[\mathbf{w}_1 \; \Lambda^{-1} \cdot \mathbf{w}_2 \; \Lambda^{-2} \cdot \mathbf{w}_3 \cdots \Lambda^{-(N-L)} \cdot \mathbf{w}_{N-L+1}]$ und $\mathbf{H}_D$ den Mittelwert der Blockmatrizen in

$$\begin{bmatrix} \mathbf{V}_1^T \\ \mathbf{V}_2^T \cdot \Lambda^{-1} \\ \mathbf{V}_3^T \cdot \Lambda^{-2} \\ \vdots \\ \mathbf{V}_L^T \cdot \Lambda^{-(L-1)} \end{bmatrix}$$

bezeichnet.

Aus den komplexwertigen Amplituden der spektralen Frequenzen lassen sich die rellen Schwingungsamplituden und die zugehörigen Phasen leicht bestimmen. Bei bekannter Geometrie des Sensorfeldes können aus diesen Werten die beobachteten, von Meßfehlern befreiten Signale rekonstruiert und an einem geeigneten Ausgabegerät, z.B. an einem Monitor, dargestellt werden. Aus den Phasen der Schwingungen und deren Verschiebungen zwischen den einzelnen Sensoren kann der Ort der Entstehung bestimmter Signalanteile sehr genau rekonstruiert werden. Hierdurch ist es beispielsweise möglich, den Herkunftsort eines pathologischen Signalanteils aus einem einzigen QRS-Komplex eines einzelnen Herzschlags im Myokard genau zu bestimmen.

In allen beschriebenen Verfahrensvarianten ist es besonders vorteilhaft, die Matrix **F** mit Hilfe der Methode der kleinsten Fehlerquadrate oder mit Hilfe der Methode der kleinsten Gesamtfehlerquadrate (total least squares) zu ermitteln. Die allgemein bekannte mathematische Literatur für Ingenieure gibt dem Fachmann die hierzu nötige Anleitung.

Die mit Hilfe des erfindungsgemäßen Verfahrens geschätzten komplexen Amplituden und Eigenwerte sind wesentlich genauer als die mit bekannten Verfahren zur Spektralanalyse bestimmten Werte. Die Schätzergebnisse des erfindungsgemäßen Verfahrens sind des weiteren wegen der nummerisch äußerst stabilen mathematischen Methoden, wie z. B. der Singulärwertzerlegung und der Eigenwertzerlegung, äußerst robust gegen Rauschsignale die den Meßsignalen überlagert sein können.

Im Falle von ungedämpften Exponentialschwingungen (verschwindende Dämpfungskonstanten) bietet die erweiterte Zustandsdarstellung eine Möglichkeit, die Schätzungsgenauigkeit zusätzlich zu verbessern. Ungedämpfte Exponentialschwingungen sind durch verschwindende Realteile der logarithmierten Eigenwerte der

Matrix F charakterisiert. In diesem Fall kann eine erweiterte Block-Hankel-Matrix

$$\begin{pmatrix} \mathbf{y}^T(0) & \mathbf{y}^T(1) & \cdots & \mathbf{y}^T(N-L) & \mathbf{y}^H(0) & \mathbf{y}^H(1) & \cdots & \mathbf{y}^H(N-L) \\ \mathbf{y}^T(1) & \mathbf{y}^T(2) & \cdots & \mathbf{y}^T(N-L+1) & \mathbf{y}^H(1) & \mathbf{y}^H(2) & \cdots & \mathbf{y}^H(N-L+1) \\ \vdots & \vdots & & \vdots & \vdots & \vdots & & \vdots \\ \mathbf{y}^T(L-1) & \mathbf{y}^T(L) & \cdots & \mathbf{y}^T(N-1) & \mathbf{y}^H(L-1) & \mathbf{y}^H(L) & \cdots & \mathbf{y}^H(N-1) \end{pmatrix}$$

eingeführt werden, welche anstelle der Matrix $\mathbf{Y}_E$ tritt. Verwendet man diese Matrix anstelle der Matrix $\mathbf{Y}_E$, so ergeben sich Schätzungen für die Frequenzen, Dämpfungskonstanten, Amplituden und Phasen der harmonischen Anteile in den Meßdaten, deren Genauigkeit, vergleichbar ist mit der Genauigkeit, die man bei linearen Prädiktionsproblemen durch gleichzeitige Kombination von Vorwärts- und Rückwärtsprädiktion erreicht. In diesem Fall ist es auch angebracht, das überbestimmte Gleichungssystem zur Bestimmung der Matrix **F** mit Hilfe eines Optimierungsverfahrens unter Verwendung von Nebenbedingungen zu lösen, welche gewährleisten, daß die logarithmierten Eigenwerte der Matrix **F** verschwindende Realteile aufweisen. Auf diese Weise erhalt man eine unitäre Matrix **F**, deren logarithmierte Eigenwerte - wie gewünscht - automatisch rein imaginär sind.

Das erfindungsgemäße Verfahren zur Spektralanalyse läßt sich nun zur Identifikation von ventrikulären Spätpoentialen in mehrkanaligen MKG- oder EKG-Daten anwenden. Diese Anwendung benutzt den Herzschlag als Trigger zur Extraktion eines relevanten Datenintervalls, dessen Fenster sich wie in Fig. 1 dargestellt, im terminalen Teil des QRS-Komplexes befindet. In einem weiteren Schritt der Vorverarbeitung ist es häufig angebracht, Gleichanteile und lineare Trends in den Meßsignalen unter Anwendung bekannter Vorverarbeitungsverfahren zu eliminieren. Zur Kompensation von Gleichanteilen und linearen Trends kann man z. B. eine Gerade an die Meßdaten anpassen und diese anschließend von den Meßdaten subtrahieren. Diese Kompensation der Gleichanteile und linearen Trends ist schematisch in Fig. 2 dargestellt. An diese Vorverarbeitungsschritte schließt sich nun die eigentliche hochauflösende Spektralanalyse an, wie sie in dieser Patentanmeldung bereits dargestellt wurde.

Fig. 1 zeigt einen typischen Verlauf des menschlichen Herzschlags. Zur Triggerung des Maximums des QRS-Komplexes steht dem Fachmann eine Reihe von bekannten Verfahren zur Verfügung. Ein hierfür besonders geeignetes Verfahren wird in der internationalen Patentanmeldung PCT DE 92/00539, angemeldet am 30.6.92, offengelegt am 18.2.93 (Erfinder: P. Strobach), beschrieben. Die in Fig. 1 definierten Parameter Verzögerung $\Delta_t$ und Fensterlänge L können (z. B. vom Arzt) frei gewählt werden. Aus allen M beobachteten Kanälen wird im ersten Schritt des erfindungsgemäßen Verfahrens der Datenausschnitt, welcher in dem durch den Paramter L (Fensterlänge) gekennzeichneten Bereich liegt, extrahiert.

Danach werden in jedem Kanal die extrahierten Datenausschnitte durch eine Gerade approximiert, z. B. mit Hilfe einer Anpassung durch Minimierung der Summe der Fehlerquadrate. Die geschätzten Geraden subtrahiert man anschließend von den extrahierten Rohdaten. Durch diese Vorgehensweise werden in allen M Kanälen möglicherweise vorhandene Gleichanteile und ggf. vorhandene lineare Trends kompensiert (s. Fig. 2).

Die Modellierung des Herzsignals im extrahierten Datenintervall als Überlagerung möglicherweise gedämpfter, sinusförmiger Schwingungen entspricht einer Schätzung der harmonischen Signalanteile im Post-QRS-Bereich des Herzschlags. Für diese Anwendung haben sich die bereits ausführlich dargestellten erfindungsgemäßen Verfahren zur hochauflösenden Mehrkanalspektralanalyse als besonders geeignet herausgestellt, da wie Experimente zeigen, alle M Sensoren des Meßaufbaus die gleichen Frequenzen registrieren, die Amplituden dieser Schwingungen und auch die Phasen dieser Schwingungen jedoch an allen Kanälen unterschiedlich sein können.

Um die bei der Singulärwertzerlegung der Blockmatrizen Y dominanten Singulärwerte bestimmen zu können, ist es zweckmäßig ein von der Energie des Einzelherzschlags unabhängiges Kriterium zu verwenden: Hierzu quadriert man die Singulärwerte und normiert diese Quadrate auf die Summe aller quadrierten Singulärwerte. Die so bestimmten relativen Energiekoeffizienten werden mit einer vorgegebenen Schwelle verglichen. Diese vorgegebene Schwelle kann mit verschiedenen statistischen Verfahren zur Signaldetektion bestimmt werden, wie sie z. B. in dem Aufsatz von M. Wax, T. Kailath, "Detection of Signals by Information Theoretic Criteria", IEEE Trans. Acoust., Speech, Signal Processing, vol. ASSP-33, pp. 387-392, April 1985, ausführlich dargestellt sind. Es ist weiterhin zweckmäßig die Schwelle so zu wählen, daß sich eine gerade Anzahl von dominanten Singulärwerten ergibt, den jede reelle sinusförmige Schwingung ist bekanntlich die Summe zweier komplexer Exponentialschwingungen.

Um die Ergebnisse des Spektralanalyseverfahrens in komfortabler Weise visuell beurteilen zu können, ist es zweckmäßig, die geschätzten harmonischen Signalanteile für jeden analysierten Herzschlag durch Überlagerung zu einem synthetischen Herzschlagsignal zusammenzusetzen und dieses synthetische Signal in geeigneter Weise unter Verwendung herkömmlicher Ausgabegeräte anzuzeigen. Diese Maßnahme ist zwar nicht unbedingt erforderlich, ermöglicht aber eine komfortable optische Auswertung der Schätzergebnisse. Auf diese Weise können Spätpotentiale sowohl räumlich als auch zeitlich im Einzelschlag lokalisiert werden.

Das erfindungsgemäße Verfahren wurde umfangreich an magnetokardiographischen Datensätzen erprobt. Die in den Figuren 3 bis 6 dargestellten Histogramme zeigen die in vier verschiedenen Datensätzen gefundenen dominanten Frequenzen. Hierbei ist die Zahl der gefundenen Frequenzen in einem Frequenzintervall über der Frequenz gemessen in Herz dargestellt. Die Verzögerung $\Delta_t$ betrug hierbei 50 ms, die Fensterlänge L betrug 90 ms. Es wurden 30 parallel arbeitende Meßkanäle ausgewertet. Bei allen vier Patienten treten deutliche Signalanteile im Bereich von 15 Hz, entsprechend dem Ausschwingen des QRS-Komplexes und 50 Hz, entsprechend der Netzfrequenz auf. Bei Patienten mit Spätpotentialen ist zusätzlich eine Häufung von harmonischen Signalanteilen um etwa 200 Hz eindeutig zu erkennen (s. Fig. 3 und 4). Diese Häufung in den oberen Frequenzbereichen ist bei gesunden Probanden nicht vorhanden (s. Fig. 4 und 5). Durch die Darstellung der gefundenen Frequenzen in Histogrammform sind Spätpotentiale deshalb sehr leicht diagnostizierbar.

Nachdem der behandelnde Arzt das Frequenzhistogramm eines Patienten begutachtet hat, kann er sich die ihn interessierenden Frequenzen im Datensatz z. B. anhand des synthetischen Signals anschauen. Hierdurch ist eine zeitliche Lokalisierung der Spätpotentiale, d. h. die Angabe, welche Spätpotentiale in welchem einzelnen Herzschlag aufgetreten sind, möglich. Durch Auswertung der Amplituden und Phasen in den M Sensorkanälen ist ferner eine räumliche Lokalisierung der Spätpotentiale möglich.

In dieser Patentanmeldung wurden die folgenden Veröffentlichungen zitiert:

D.E. Balderson, et. al., "The detection, significance and effect of drugs upon ventricular late potentials", Automedia, vol. 13, pp. 67-96, 1991.

E.J. Berbari, R. Lazzara, "An Introduction to High-Resolution ECG Recordings of Cardiac Late Potentials", Arch. Intern. med., vol. 148, pp. 1859-1863, August 1988.

S.M. Kay, S.L. Marple, "Spectrum Analysis - A Modern Perspective", Proceedings of the IEEE, Vol. 69, No. 11, November 1981.

B.D. Rao, K.S. Arun, "Model based Processing of Signals: A State Space Approach", Proc. IEEE, vol. 80, pp. 283-309, Feb. 1992.

S. Haykin, Editor, "Nonlinear Methods of Spectral Analysis", Topics in Applied Physics, vol. 34, Springer-Verlag, 1979.

Internationale Patentanmeldung PCT DE 92/00539, angemeldet am 30.6.92, offengelegt am 18.2.93 (Erfinder: P. Strobach)

M. Wax, T. Kailath, "Detection of Signals by Information Theoretic Criteria", IEEE Trans. Acoust., Speech, Signal Processing, vol. ASSP-33, pp. 387-392, April 1985.

## Patentansprüche

1. Spektralanalyseverfahren zur mehrkanaligen hochauflösenden Spektralanalyse, dadurch gekennzeichnet, daß

   a) M zeitliche Folgen $y_i(n)$ von Meßdaten der Sensorkanäle $1 \leqq i \leqq M$, mit $M > 1$, zu den Zeitpunkten $0 \leqq n \leqq N-1$ beobachtet und zu einer Matrix $\mathbf{Y}_E$ der Form

$$\mathbf{Y}_E = \begin{pmatrix} \mathbf{y}^T(0) & \mathbf{y}^T(1) & \cdots & \mathbf{y}^T(N-L) \\ \mathbf{y}^T(1) & \mathbf{y}^T(2) & \cdots & \mathbf{y}^T(N-L+1) \\ \vdots & \vdots & & \vdots \\ \mathbf{y}^T(L-1) & \mathbf{y}^T(L) & \cdots & \mathbf{y}^T(N-1) \end{pmatrix}$$

   mit $\mathbf{y}(n) = (y_1(n),...,y_M(n))^T$ zusammengefaßt werden,

   b) eine Singulärwertzerlegung $\mathbf{Y}_E = \mathbf{U}\cdot\Sigma\cdot\mathbf{V}^H$ der Matrix $\mathbf{Y}_E$ durchgeführt wird, und die Singulärwerte einer Schwellwertoperation unterzogen werden, wodurch alle Singulärwerte von $\mathbf{Y}_E$, die dem Betrage nach kleiner sind als der Schwellwert, durch Null ersetzt werden und wonach alle p dominanten, d.h. nicht durch Null ersetzten, Singulärwerte von $\mathbf{Y}_E$ in der Blockmatrix $\Sigma_1$ der Zerlegung

$$\mathbf{Y}_E = (\mathbf{U}_1 \quad \mathbf{U}_2) \cdot \begin{pmatrix} \mathbf{\Sigma}_1 & \mathbf{0} \\ \mathbf{0} & \mathbf{0} \end{pmatrix} \cdot \begin{pmatrix} \mathbf{V}_1^H \\ \mathbf{V}_2^H \end{pmatrix}$$

enthalten sind,
c) diejenigen Matrizen $\Theta_1$ und $\Theta_2$ bestimmt werden, für die

$$\mathbf{U}_1 \cdot \mathbf{\Sigma}_1^{1/2} = \begin{pmatrix} \Theta_1 \\ \theta_2 \end{pmatrix} = \begin{pmatrix} \theta_1 \\ \Theta_2 \end{pmatrix}$$

gilt, wobei $\theta_1$ und $\theta_2$ jeweils die erste bzw. die letzte Blockmatrix bezeichnen,
d) eine Matrix **F** bestimmt wird, die eine optimale Lösung des im allgemeinen überbestimmten Gleichungssystems $\Theta_1 \cdot \mathbf{F} = \Theta_2$ ist, wobei die Imaginärteile der natürlichen Logarithmen der Eigenwerte von **F** die gesuchten spektralen Frequenzen und die Realteile der natürlichen Logarithmen der Eigenwerte von **F** mit diesen Frequenzen assoziierte Dämpfungskonstanten sind.

**2.** Spektralanalyseverfahren zur mehrkanaligen hochauflösenden Spektralanalyse, bei dem M zeitliche Folgen $y_i(n)$ von Meßdaten der Sensorkanäle $\mathbf{1} \leqq i \leqq M$, mit $\mathbf{M} > 1$, zu den Zeitpunkten $\mathbf{0} \leqq n \leqq N\mathbf{-1}$ beobachtet werden, dadurch gekennzeichnet, daß spektrale Frequenzen und mit diesen Frequenzen assoziierte Dämpfungskonstanten als Imaginär- bzw. Realteile der natürlichen Logarithmen der Eigenwerte einer Matrix **F** bestimmt werden, die als optimale Lösung eines im allgemeinen überbestimmten Gleichungssystems $\Theta_1 \cdot \mathbf{F} = \Theta_2$ definiert ist, wobei die Matrizen $\Theta_1$ und $\Theta_2$ definiert sind durch die Beziehung

$$\mathbf{U}_1 \cdot \mathbf{\Sigma}_1^{1/2} = \begin{pmatrix} \Theta_1 \\ \theta_2 \end{pmatrix} = \begin{pmatrix} \theta_1 \\ \Theta_2 \end{pmatrix},$$

in der $\theta_1$ und $\theta_2$ jeweils die erste bzw. die letzte Blockmatrix bezeichnen, und in der die Matrizen $\Sigma_1$ und $\mathbf{U}_1$ definiert sind durch eine approximative Singulärwertzerlegung

$$\mathbf{Y}_E = (\mathbf{U}_1 \quad \mathbf{U}_2) \cdot \begin{pmatrix} \mathbf{\Sigma}_1 & \mathbf{0} \\ \mathbf{0} & \mathbf{0} \end{pmatrix} \cdot \begin{pmatrix} \mathbf{V}_1^H \\ \mathbf{V}_2^H \end{pmatrix}$$

einer Matrix

$$\mathbf{Y}_E = \begin{pmatrix} \mathbf{y}^T(0) & \mathbf{y}^T(1) & \cdots & \mathbf{y}^T(N-L) \\ \mathbf{y}^T(1) & \mathbf{y}^T(2) & \cdots & \mathbf{y}^T(N-L+1) \\ \vdots & \vdots & & \vdots \\ \mathbf{y}^T(L-1) & \mathbf{y}^T(L) & \cdots & \mathbf{y}^T(N-1) \end{pmatrix},$$

wobei $\mathbf{y}(n) = (y_1(n),...,y_M(n))^T$, und wobei die approximative Singulärwertzerlegung aus einer ursprünglichen Singulärwertzerlegung $\mathbf{Y}_E = \mathbf{U} \cdot \Sigma \cdot \mathbf{V}^H$ dadurch ermittelt wird, daß die Singulärwerte einer Schwellwertoperation unterzogen werden, wodurch alle Singulärwerte von $\mathbf{Y}_E$, die dem Betrage nach kleiner sind als der Schwellwert, durch Null ersetzt werden und wonach alle p dominanten, d.h. nicht durch Null ersetzten, Singulärwerte von $\mathbf{Y}_E$ in der Blockmatrix $\Sigma_1$ der approximativen Zerlegung enthalten sind.

**3.** Spektralanalyseverfahren zur mehrkanaligen hochauflösenden Spektralanalyse, dadurch gekennzeichnet, daß
a) M zeitliche Folgen $y_i(n)$ von Meßdaten der Sensorkanäle $\mathbf{1} \leqq i \leqq M$, mit $\mathbf{M} > 1$, zu den Zeitpunkten -

$\mathbf{0 \leqq n \leqq N\text{-}1}$ beobachtet und zu einer Matrix $\mathbf{Y}_T$ der Form

$$\mathbf{Y}_T = \begin{pmatrix} \mathbf{y}(0) & \mathbf{y}(1) & \cdots & \mathbf{y}(N-L) \\ \mathbf{y}(1) & \mathbf{y}(2) & \cdots & \mathbf{y}(N-L+1) \\ \vdots & \vdots & & \vdots \\ \mathbf{y}(L-1) & \mathbf{y}(L) & \cdots & \mathbf{y}(N-1) \end{pmatrix}$$

mit $\mathbf{y}(n) = (y_1(n),...,y_M(n))^T$ zusammengefaßt werden,
b) eine Singulärwertzerlegung $\mathbf{Y}_T = \mathbf{U}\cdot\Sigma\cdot\mathbf{V}^H$ der Matrix $\mathbf{Y}_T$ durchgeführt wird, und die Singulärwerte einer Schwellwertoperation unterzogen werden, wodurch alle Singulärwerte von $\mathbf{Y}_T$, die dem Betrage nach kleiner sind als der Schwellwert, durch Null ersetzt werden und wonach alle p dominanten, d.h. nicht durch Null ersetzten, Singulärwerte von $\mathbf{Y}_T$ in der Blockmatrix $\Sigma_1$ der Zerlegung

$$\mathbf{Y}_T = (\mathbf{U}_1 \quad \mathbf{U}_2)\cdot\begin{pmatrix} \boldsymbol{\Sigma}_1 & \mathbf{0} \\ \mathbf{0} & \mathbf{0} \end{pmatrix}\cdot\begin{pmatrix} \mathbf{V}_1^H \\ \mathbf{V}_2^H \end{pmatrix}$$

enthalten sind,
c) diejenigen Matrizen $\Theta_1$ und $\Theta_2$ bestimmt werden, für die

$$\mathbf{U}_1 \cdot \boldsymbol{\Sigma}_1^{1/2} = \begin{pmatrix} \Theta_1 \\ \theta_2 \end{pmatrix} = \begin{pmatrix} \theta_1 \\ \Theta_2 \end{pmatrix}$$

gilt, wobei $\theta_1$ und $\theta_2$ jeweils die erste bzw. die letzte Blockmatrix bezeichnen,
d) eine Matrix **F** bestimmt wird, die eine optimale Lösung des im allgemeinen überbestimmten Gleichungssystems $\Theta_1\cdot\mathbf{F} = \Theta_2$ ist, wobei die Imaginärteile der natürlichen Logarithmen der Eigenwerte von **F** die gesuchten spektralen Frequenzen und die Realteile der natürlichen Logarithmen der Eigenwerte von **F** mit diesen Frequenzen assoziierte Dämpfungskonstanten sind.

4. Spektralanalyseverfahren zur mehrkanaligen hochauflösenden Spektralanalyse, bei dem M zeitliche Folgen $y_i(n)$ von Meßdaten der Sensorkanäle $\mathbf{1 \leqq i \leqq M}$, mit $\mathbf{M} > 1$, zu den Zeitpunkten $\mathbf{0 \leqq n \leqq N\text{-}1}$ beobachtet werden, dadurch gekennzeichnet, daß spektrale Frequenzen und mit diesen Frequenzen assoziierte Dämpfungskonstanten als Imaginär- bzw. Realteile der natürlichen Logarithmen der Eigenwerte einer Matrix **F** bestimmt werden, die als optimale Losung eines im allgemeinen überbestimmten Gleichungssystems $\Theta_1\cdot\mathbf{F} = \Theta_2$ definiert ist, wobei die Matrizen $\Theta_1$ und $\Theta_2$ definiert sind durch die Beziehung

$$\mathbf{U}_1 \cdot \boldsymbol{\Sigma}_1^{1/2} = \begin{pmatrix} \Theta_1 \\ \theta_2 \end{pmatrix} = \begin{pmatrix} \theta_1 \\ \Theta_2 \end{pmatrix},$$

in der $\theta_1$ und $\theta_2$ jeweils die erste bzw. die letzte Blockmatrix bezeichnen, und in der die Matrizen $\Sigma_1$ und $U_1$ definiert sind durch eine approximative Singulärwertzerlegung

$$\mathbf{Y}_T = (\mathbf{U}_1 \quad \mathbf{U}_2)\cdot\begin{pmatrix} \boldsymbol{\Sigma}_1 & \mathbf{0} \\ \mathbf{0} & \mathbf{0} \end{pmatrix}\cdot\begin{pmatrix} \mathbf{V}_1^H \\ \mathbf{V}_2^H \end{pmatrix}$$

einer Matrix

$$\mathbf{Y}_T = \begin{pmatrix} \mathbf{y}(0) & \mathbf{y}(1) & \cdots & \mathbf{y}(N-L) \\ \mathbf{y}(1) & \mathbf{y}(2) & \cdots & \mathbf{y}(N-L+1) \\ \vdots & \vdots & & \vdots \\ \mathbf{y}(L-1) & \mathbf{y}(L) & \cdots & \mathbf{y}(N-1) \end{pmatrix},$$

wobei $\mathbf{y}(n) = (y_1(n),...,y_M(n))^T$, und wobei die approximative Singulärwertzerlegung aus einer ursprünglichen Singulärwertzerlegung $\mathbf{Y}_T = \mathbf{U}\cdot\Sigma\cdot\mathbf{V}^H$ dadurch ermittelt wird, daß die Singulärwerte einer Schwellwertoperation unterzogen werden, wodurch alle Singulärwerte von $\mathbf{Y}_T$, die dem Betrage nach kleiner sind als der Schwellwert, durch Null ersetzt werden und wonach alle p dominanten, d.h. nicht durch Null ersetzten, Singulärwerte von $\mathbf{Y}_T$ in der Blockmatrix $\Sigma_1$ der approximativen Zerlegung enthalten sind.

**5.** Verfahren nach einem der Ansprüche 1 oder 2, dadurch gekennzeichnet, daß die Matrix **C** der komplexwertigen Amplituden $c_{j,i}$ der mit den spektralen Frequenzen j = 1,...,p assoziierten Schwingungen im Sensorkanal i = 1,...,M durch die Ausführung der folgenden Schritte ermittelt wird:

a) ausgehend von der Ähnlichkeitstransformation $\mathbf{F} = \mathbf{S}\cdot\Lambda\cdot\mathbf{S}^{-1}$ der Matrix **F** und deren Diagonalform $\Lambda$ werden die Matrizen

$$\mathbf{U}_1\cdot\Sigma_1^{1/2}\cdot\mathbf{S} \quad \text{und} \quad \mathbf{S}^{-1}\cdot\Sigma_1^{1/2}\cdot\mathbf{V}_1^H$$

aufgestellt und in Blockmatrizen

$$\mathbf{U}_1\cdot\Sigma_1^{1/2}\cdot\mathbf{S} = \begin{bmatrix} \mathbf{v}_1^T \\ \mathbf{v}_2^T \\ \mathbf{v}_3^T \\ \vdots \\ \mathbf{v}_L^T \end{bmatrix} \quad \text{sowie} \quad \mathbf{S}^{-1}\cdot\Sigma_1^{1/2}\cdot\mathbf{V}_1^H = [\mathbf{W}_1 \quad \mathbf{W}_2 \quad \mathbf{W}_3 \quad \cdots \quad \mathbf{W}_{N-L+1}]$$

unterteilt;

b) aus diesen Darstellungen werden die Matrizen

$$\begin{bmatrix} \mathbf{v}_1^T \\ \mathbf{v}_2^T\cdot\Lambda^{-1} \\ \mathbf{v}_3^T\cdot\Lambda^{-2} \\ \vdots \\ \mathbf{v}_L^T\cdot\Lambda^{-(L-1)} \end{bmatrix}$$

sowie

$$[\mathbf{W}_1 \ \Lambda^{-1}\cdot\mathbf{W}_2 \ \Lambda^{-2}\cdot\mathbf{W}_3 \cdots \Lambda^{-(N-L)}\cdot\mathbf{W}_{N-L+1}]$$

abgeleitet;

c) die Matrix **C** der komplexen Amplituden wird dann nach der Beziehung $\mathbf{C} = \boldsymbol{diag}\{h_1,h_2,...,h_p\}\cdot\mathbf{X}_D$ ermittelt, wobei $\mathbf{h}^T = (h_1,h_2,...,h_p)$ den Mittelwert der Zeilenvektoren von

$$\begin{bmatrix} \mathbf{v}_1^T \\ \mathbf{v}_2^T \cdot \Lambda^{-1} \\ \mathbf{v}_3^T \cdot \Lambda^{-2} \\ \vdots \\ \mathbf{v}_L^T \cdot \Lambda^{-(L-1)} \end{bmatrix}$$

und $\mathbf{X}_D$ den Mittelwert der Blockmatrizen in $[\mathbf{W}_1\ \Lambda^{-1}\cdot\mathbf{W}_2\ \Lambda^{-2}\cdot\mathbf{W}_3 \cdots \Lambda^{-(N-L)}\cdot\mathbf{W}_{N-L+1}]$ bezeichnet.

**6.** Verfahren nach einem der Ansprüche 3 oder 4, dadurch gekennzeichnet, daß die Matrix **C** der komplexwertigen Amplituden $c_{j,i}$ der mit den spektralen Frequenzen j = 1,...,p assoziierten Schwingungen im Sensorkanal i = 1,...,M durch die Ausführung der folgenden Schritte ermittelt wird:

a) ausgehend von der Ähnlichkeitstransformation $\mathbf{F} = \mathbf{S}\cdot\Lambda\cdot\mathbf{S}^{-1}$ der Matrix **F** und deren Diagonalform $\Lambda$ werden die Matrizen

$$\mathbf{U}_1 \cdot \Sigma_1^{1/2} \cdot \mathbf{S} \quad \text{und} \quad \mathbf{S}^{-1} \cdot \Sigma_1^{1/2} \cdot \mathbf{V}_1^H$$

aufgestellt und in Blockmatrizen

$$\mathbf{U}_1 \cdot \Sigma_1^{1/2} \cdot \mathbf{S} = \begin{bmatrix} \mathbf{V}_1^T \\ \mathbf{V}_2^T \\ \mathbf{V}_3^T \\ \vdots \\ \mathbf{V}_L^T \end{bmatrix} \quad \text{sowie} \quad \mathbf{S}^{-1} \cdot \Sigma_1^{1/2} \cdot \mathbf{V}_1^H = [\mathbf{w}_1 \quad \mathbf{w}_2 \quad \mathbf{w}_3 \quad \cdots \quad \mathbf{w}_{N-L+1}]$$

unterteilt;

b) aus diesen Darstellungen werden die Matrizen

$$\begin{bmatrix} \mathbf{V}_1^T \\ \mathbf{V}_2^T \cdot \Lambda^{-1} \\ \mathbf{V}_3^T \cdot \Lambda^{-2} \\ \vdots \\ \mathbf{V}_L^T \cdot \Lambda^{-(L-1)} \end{bmatrix}$$

sowie

$$[\mathbf{w}_1\ \Lambda^{-1}\cdot\mathbf{w}_2\ \Lambda^{-2}\cdot\mathbf{w}_3 \cdots \Lambda^{-(N-L)}\cdot\mathbf{w}_{N-L+1}]$$

abgeleitet;

c) die Matrix **C** der komplexen Amplituden wird dann nach der Beziehung $\mathbf{C}^T = \mathbf{H}_D\cdot\mathbf{diag}\{x_1,x_2,...,x_p\}$ ermittelt, wobei $\mathbf{x} = (x_1,x_2,...,x_p)^T$ den Mittelwert der Spaltenvektoren von $[\mathbf{w}_1\ \Lambda^{-1}\cdot\mathbf{w}_2\ \Lambda^{-2}\cdot\mathbf{w}_3 \cdots \Lambda^{-(N-L)}\cdot\mathbf{w}_{N-L+1}]$ und $\mathbf{H}_D$ den Mittelwert der Blockmatrizen in

$$\begin{bmatrix} \mathbf{v}_1^T \\ \mathbf{v}_2^T \cdot \Lambda^{-1} \\ \mathbf{v}_3^T \cdot \Lambda^{-2} \\ \vdots \\ \mathbf{v}_L^T \cdot \Lambda^{-(L-1)} \end{bmatrix}$$

bezeichnet.

7. Verfahren nach einem der vorhergehenden Ansprüche, bei dem die Matrix **F** mit Hilfe der Methode der kleinsten Fehlerquadrate ermittelt wird.

8. Verfahren nach einem der vorhergehenden Ansprüche, bei dem die Matrix **F** mit Hilfe der Methode der kleinsten Gesamtfehlerquadrate (total least squares) ermittelt wird.

9. Verfahren nach einem der Ansprüche 1 oder 2, bei dem sämtliche Dämpfungskonstanten Null sind und anstelle der Matrix $\mathbf{Y}_E$ die Matrix

$$\begin{pmatrix} \mathbf{y}^T(0) & \mathbf{y}^T(1) & \cdots & \mathbf{y}^T(N-L) & \mathbf{y}^H(0) & \mathbf{y}^H(1) & \cdots & \mathbf{y}^H(N-L) \\ \mathbf{y}^T(1) & \mathbf{y}^T(2) & \cdots & \mathbf{y}^T(N-L+1) & \mathbf{y}^H(1) & \mathbf{y}^H(2) & \cdots & \mathbf{y}^H(N-L+1) \\ \vdots & \vdots & & \vdots & \vdots & \vdots & & \vdots \\ \mathbf{y}^T(L-1) & \mathbf{y}^T(L) & \cdots & \mathbf{y}^T(N-1) & \mathbf{y}^H(L-1) & \mathbf{y}^H(L) & \cdots & \mathbf{y}^H(N-1) \end{pmatrix}$$

verwendet wird.

10. Verfahren nach Anspruch 9, bei dem die Matrix **F** unter der Nebenbedingung ermittelt wird, daß alle Eigenwerte vom Betrag eins sind.

11. Verfahren nach einem der vorhergehenden Ansprüche, dadurch gekennzeichnet, daß dasselbe zur Detektion von ventrikulären Spätpotentialen in mehrkanaligen medizintechnischen Meßverfahren verwendet wird.

12. Verwendung nach Anspruch 11, dadurch gekennzeichnet, daß die Meßdaten durch mehrkanalige, zeitliche Abtastung vom Herzen stammender Signale im terminalen Anteil des QRS-Komplexes und in der ST-Strecke gewonnen werden.

13. Verwendung nach Anspruch 12, dadurch gekennzeichnet, daß vor der Durchführung der hochauflösenden Spektralanalyse Gleichanteile und lineare Trends in den Meßdaten kompensiert werden.

14. Verwendung nach einem der Ansprüche 11 bis 13 in Verbindung mit einem der Ansprüche 5 oder 6, dadurch gekennzeichnet, daß ein Sensorarray mit definierter räumlicher Anordnung der einzelnen Sensoren verwendet wird, und daß aus den komplexen Amplituden oder, dazu gleichwertig, aus den reellen Amplituden und Phasen der Schwingungen auf der Grundlage der bekannten räumlichen Anordnung der einzelnen Sensoren die räumliche Lage einer Quelle bestimmter Signalanteile ermittelt wird.

15. Verwendung nach Anspruch 14, bei der pathologische Regionen im Herzmuskel, die mit der Entstehung von Spätpotentialen zusammenhängen, lokalisiert werden.

16. Verwendung nach einem der vorhergehenden Ansprüche in Verbindung mit einem der Ansprüche 5 oder 6 zur Visualisierung der harmonischen Anteile in den Meßdaten, dadurch gekennzeichnet, daß ein synthetisches Signal, welches durch phasenrichtige Überlagerung der ermittelten Schwingungen erzeugt wird, auf einem Ausgabegerät dargestellt wird.

**17.** Elektronisches Datenverarbeitungssystem zur Durchführung einer mehrkanaligen hochauflösenden Spektralanalyse mit einem Verfahren nach einem der Ansprüche 1 bis 10.

## Claims

**1.** Method of spectral analysis for multichannel high-resolution spectral analysis, characterized in that
a) M chronological sequences $y_i(n)$ of measured data of the sensor channels $1 \leqq i \leqq M$, with M>1, are observed at the instants $0 \leqq n \leqq N-1$ and are combined into a matrix $\mathbf{Y}_E$ of the formula

$$\mathbf{Y}_E = \begin{pmatrix} \mathbf{y}^T(0) & \mathbf{y}^T(1) & \cdots & \mathbf{y}^T(N-L) \\ \mathbf{y}^T(1) & \mathbf{y}^T(2) & \cdots & \mathbf{y}^T(N-L+1) \\ \vdots & \vdots & & \vdots \\ \mathbf{y}^T(L-1) & \mathbf{y}^T(L) & \cdots & \mathbf{y}^T(N-1) \end{pmatrix}$$

where $\mathbf{y}(n) = (y_1(n),...,y_M(n))^T$,
b) a singular value resolution $\mathbf{Y}_E = \mathbf{U}\cdot\Sigma\cdot\mathbf{V}^H$ of the matrix $\mathbf{Y}_E$ is carried out, and the singular values are subjected to a threshold value operation, whereby all singular values of $\mathbf{Y}_E$ which in terms of magnitude are smaller than the threshold value, are replaced by zero and according to which all p singular values of $\mathbf{Y}_E$ which are dominant, ie. not replaced by zero, are included in the block matrix $\Sigma_1$ of the resolution

$$\mathbf{Y}_E = (\mathbf{U}_1 \quad \mathbf{U}_2)\cdot\begin{pmatrix} \Sigma_1 & \mathbf{0} \\ \mathbf{0} & \mathbf{0} \end{pmatrix}\cdot\begin{pmatrix} \mathbf{V}_1^H \\ \mathbf{V}_2^H \end{pmatrix}$$

c) those matrices $\theta_1$ and $\theta_2$ are determined for which

$$\mathbf{U}_1\cdot\Sigma_1^{1/2} = \begin{pmatrix} \Theta_1 \\ \theta_2 \end{pmatrix} = \begin{pmatrix} \theta_1 \\ \Theta_2 \end{pmatrix}$$

is applicable, where $\theta_1$ and $\theta_2$ designate in each instance the first and respectively the last block matrix,
d) a matrix **F** is determined, which is an optimal solution of the system of equations $\theta_1\cdot\mathbf{F} = \theta_2$ which is in general defined with redundancy, the imaginary parts of the natural logarithms of the eigenvalues of **F** being the sought spectral frequencies and the real parts of the natural logarithms of the eigenvalues of **F** being attenuation constants associated with these frequencies.

**2.** Method of spectral analysis for multichannel high-resolution spectral analysis, in which M chronological sequences $y_i(n)$ of measured data of the sensor channels $1 \leqq i \leqq M$, with M>1, are observed at the instants $0 \leqq n \leqq N-1$ characterized in that spectral frequencies and attenuation constants associated with these frequencies are determined as imaginary and respectively real parts of the natural logarithms of the eigenvalues of a matrix **F**, which is defined as optimal solution of a system of equations $\theta_1\cdot\mathbf{F} = \theta_2$ which is in general defined with redundancy, the matrices $\theta_1$ and $\theta_2$ being defined by the relation

$$\mathbf{U}_1\cdot\Sigma_1^{1/2} = \begin{pmatrix} \Theta_1 \\ \theta_2 \end{pmatrix} = \begin{pmatrix} \theta_1 \\ \Theta_2 \end{pmatrix},$$

in which $\theta_1$ and $\theta_2$ designate in each instance the first and respectively the last block matrix, and in which the matrices $\Sigma_1$ and $\mathbf{U}_1$ are defined by an approximate singular value resolution

$$\mathbf{Y}_E = (\mathbf{U}_1 \quad \mathbf{U}_2) \cdot \begin{pmatrix} \boldsymbol{\Sigma}_1 & \mathbf{0} \\ \mathbf{0} & \mathbf{0} \end{pmatrix} \cdot \begin{pmatrix} \mathbf{V}_1^H \\ \mathbf{V}_2^H \end{pmatrix}$$

of a matrix

$$\mathbf{Y}_E = \begin{pmatrix} \mathbf{y}^T(0) & \mathbf{y}^T(1) & \cdots & \mathbf{y}^T(N-L) \\ \mathbf{y}^T(1) & \mathbf{y}^T(2) & \cdots & \mathbf{y}^T(N-L+1) \\ \vdots & \vdots & & \vdots \\ \mathbf{y}^T(L-1) & \mathbf{y}^T(L) & \cdots & \mathbf{y}^T(N-1) \end{pmatrix},$$

where $\mathbf{y}(n) = (y_1(n),...,y_M(n))^T$, and where the approximate singular value resolution is determined from an original singular value resolution $\mathbf{Y}_E = \mathbf{U}\cdot\Sigma\cdot\mathbf{V}^H$ in that the singular values are subjected to a threshold value operation, whereby all singular values of $\mathbf{Y}_E$ which in terms of magnitude are smaller than the threshold value are replaced by zero and according to which all p singular values of $\mathbf{Y}_E$ which are dominant, ie. not replaced by zero, are included in the block matrix $\Sigma_1$ of the approximate resolution.

**3.** Method for spectral analysis for multichannel high-resolution spectral analysis, characterized in that

a) M chronological sequences $y_i(n)$ of measured data of the sensor channels $1 \leqq i \leqq M$, with M>1, are observed at the instants $0 \leqq n \leqq N-1$ and are combined into a matrix $\mathbf{Y}_T$ of the form

$$\mathbf{Y}_T = \begin{pmatrix} \mathbf{y}(0) & \mathbf{y}(1) & \cdots & \mathbf{y}(N-L) \\ \mathbf{y}(1) & \mathbf{y}(2) & \cdots & \mathbf{y}(N-L+1) \\ \vdots & \vdots & & \vdots \\ \mathbf{y}(L-1) & \mathbf{y}(L) & \cdots & \mathbf{y}(N-1) \end{pmatrix}$$

where $\mathbf{y}(n) = (y_1(n),...,y_M(n))^T$,

b) a singular value resolution $\mathbf{Y}_T = \mathbf{U}\cdot\Sigma\cdot\mathbf{V}^H$ of the matrix $\mathbf{Y}_T$ is carried out, and the singular values are subjected to a threshold value operation, whereby all singular values of $\mathbf{Y}_T$ which in terms of magnitude are smaller than the threshold value are replaced by zero and according to which all p singular values are $\mathbf{Y}_T$ which are dominant, ie. not replaced by zero, are included in the block matrix $\Sigma_1$ of the resolution

$$\mathbf{Y}_T = (\mathbf{U}_1 \quad \mathbf{U}_2) \cdot \begin{pmatrix} \boldsymbol{\Sigma}_1 & \mathbf{0} \\ \mathbf{0} & \mathbf{0} \end{pmatrix} \cdot \begin{pmatrix} \mathbf{V}_1^H \\ \mathbf{V}_2^H \end{pmatrix},$$

c) those matrices $\theta_1$ and $\theta_2$ are determined for which

$$\mathbf{U}_1 \cdot \boldsymbol{\Sigma}_1^{1/2} = \begin{pmatrix} \Theta_1 \\ \theta_2 \end{pmatrix} = \begin{pmatrix} \theta_1 \\ \Theta_2 \end{pmatrix}$$

is applicable, where $\theta_1$ and $\theta_2$ designate in each instance the first and respectively the last block matrix,

d) a matrix $\mathbf{F}$ is determined, which is an optimal solution of the system of equations $\theta_1\cdot\mathbf{F} = \theta_2$ which is in general defined with redundancy, the imaginary parts of the natural logarithms of the eigenvalues of $\mathbf{F}$ being the sought spectral frequencies and the real parts of the natural logarithms of the eigenvalues of $\mathbf{F}$ being attenuation constants associated with these frequencies.

**4.** Method of spectral analysis for multichannel high-resolution spectral analysis, in which M chronological sequences $y_i(n)$ of measured data of the sensor channels $1 \leqq i \leqq M$, with M>1, are observed at the instants $0 \leqq n \leqq N-1$, characterized in that spectral frequencies and attenuation constants associated with these frequencies are determined as imaginary and respectively real parts of the natural logarithms of the eigenvalues of a matrix **F**, which is defined as optimal solution of a system of equations $\theta_1 \cdot \mathbf{F} = \theta_2$ which is in general defined with redundancy, the matrices $\theta_1$ and $\theta_2$ being defined by the relation

$$\mathbf{U}_1 \cdot \mathbf{\Sigma}_1^{1/2} = \begin{pmatrix} \Theta_1 \\ \theta_2 \end{pmatrix} = \begin{pmatrix} \theta_1 \\ \Theta_2 \end{pmatrix} ,$$

in which $\theta_1$ and $\theta_2$ designate in each instance the first and respectively the last block matrix, and in which the matrices $\Sigma_1$ and $\mathbf{U}_1$ are defined by an approximate singular value resolution

$$\mathbf{Y}_T = (\mathbf{U}_1 \quad \mathbf{U}_2) \cdot \begin{pmatrix} \mathbf{\Sigma}_1 & \mathbf{0} \\ \mathbf{0} & \mathbf{0} \end{pmatrix} \cdot \begin{pmatrix} \mathbf{V}_1^H \\ \mathbf{V}_2^H \end{pmatrix}$$

of a matrix

$$\mathbf{Y}_T = \begin{pmatrix} \mathbf{y}(0) & \mathbf{y}(1) & \cdots & \mathbf{y}(N-L) \\ \mathbf{y}(1) & \mathbf{y}(2) & \cdots & \mathbf{y}(N-L+1) \\ \vdots & \vdots & & \vdots \\ \mathbf{y}(L-1) & \mathbf{y}(L) & \cdots & \mathbf{y}(N-1) \end{pmatrix} ,$$

where $\mathbf{y}(n) = (y_1(n),...,y_M(n))^T$, and where the approximate singular value resolution is determined from an original singular value resolution $\mathbf{Y}_T = \mathbf{U} \cdot \Sigma \cdot \mathbf{V}^H$ in that the singular values are subjected to a threshold value operation, whereby all singular values of $\mathbf{Y}_T$ which in terms of magnitude are smaller than the threshold value, are replaced by zero and according to which all p singular values of $\mathbf{Y}_T$ which are dominant, ie. not replaced by zero, are included in the block matrix $\Sigma_1$ of the approximate resolution.

**5.** Method according to one of Claims 1 or 2, characterized in that the matrix **C** of the complex-value amplitudes $c_{j,i}$ of the oscillations, associated with the spectral frequencies j = 1,...,p, in the sensor channel i = 1,...,M is determined by the execution of the following steps:
a) proceeding from the similarity transformation $\mathbf{F} = \mathbf{S} \cdot \Lambda \cdot \mathbf{S}^{-1}$ of the matrix **F** and its diagonal form $\Lambda$, the matrices

$$\mathbf{U}_1 \cdot \mathbf{\Sigma}_1^{1/2} \cdot \mathbf{S} \quad \text{and} \quad \mathbf{S}^{-1} \cdot \mathbf{\Sigma}_1^{1/2} \cdot \mathbf{V}_1^H$$

are set up and subdivided into block matrices

$$\mathbf{U}_1 \cdot \mathbf{\Sigma}_1^{1/2} \cdot \mathbf{S} = \begin{bmatrix} \mathbf{v}_1^T \\ \mathbf{v}_2^T \\ \mathbf{v}_3^T \\ \vdots \\ \mathbf{v}_L^T \end{bmatrix} \quad \text{as well as} \quad \mathbf{S}^{-1} \cdot \mathbf{\Sigma}_1^{1/2} \cdot \mathbf{V}_1^H = [\mathbf{w}_1 \quad \mathbf{w}_2 \quad \mathbf{w}_3 \quad \cdots \quad \mathbf{w}_{N-L+1}] ;$$

b) the matrices

$$\begin{bmatrix} \mathbf{v}_1^T \\ \mathbf{v}_2^T \cdot \Lambda^{-1} \\ \mathbf{v}_3^T \cdot \Lambda^{-2} \\ \vdots \\ \mathbf{v}_L^T \cdot \Lambda^{-(L-1)} \end{bmatrix}$$

as well as

$$[\mathbf{W}_1\ \Lambda^{-1} \cdot \mathbf{W}_2\ \Lambda^{-2} \cdot \mathbf{W}_3 \cdots \Lambda^{-(N-L)} \cdot \mathbf{W}_{N-L+1}]$$

are derived from these representations;

c) the matrix **C** of the complex amplitudes is then determined in accordance with the relation $\mathbf{C}=diag\{h_1,h_2,\dots,h_p\} \cdot \mathbf{X}_D$, where $\mathbf{h}^T = (h_1,h_2,\dots,h_p)$ designates the mean value of the row vectors of

$$\begin{bmatrix} \mathbf{v}_1^T \\ \mathbf{v}_2^T \cdot \Lambda^{-1} \\ \mathbf{v}_3^T \cdot \Lambda^{-2} \\ \vdots \\ \mathbf{v}_L^T \cdot \Lambda^{-(L-1)} \end{bmatrix}$$

and $\mathbf{X}_D$ designates the mean value of the block matrices in $[\mathbf{W}_1\ \Lambda^{-1} \cdot \mathbf{W}_2\ \Lambda^{-2} \cdot \mathbf{W}_3 \cdots \Lambda^{-(N-L)} \cdot \mathbf{W}_{N-L+1}]$.

**6.** Method according to one of Claims 3 or 4, characterized in that the matrix **C** of the complex-value amplitudes $\mathbf{c}_{j,i}$ of the oscillations, associated with the spectral frequencies j = 1,...,p, in the sensor channel i = 1,...,**M** is determined by the execution of the following steps:

a) proceeding from the similarity transformation $\mathbf{F} = \mathbf{S} \cdot \Lambda \cdot \mathbf{S}^{-1}$ of the matrix **F** and its diagonal form $\Lambda$, the matrices

$$\mathbf{U}_1 \cdot \Sigma_1^{1/2} \cdot \mathbf{S} \text{ and } \mathbf{S}^{-1} \cdot \Sigma_1^{1/2} \cdot \mathbf{V}_1^H$$

are set up and are subdivided into block matrices

$$\mathbf{U}_1 \cdot \Sigma_1^{1/2} \cdot \mathbf{S} = \begin{bmatrix} \mathbf{v}_1^T \\ \mathbf{v}_2^T \\ \mathbf{v}_3^T \\ \vdots \\ \mathbf{v}_L^T \end{bmatrix} \text{ as well as } \mathbf{S}^{-1} \cdot \Sigma_1^{1/2} \cdot \mathbf{V}_1^H = [\mathbf{W}_1\ \ \mathbf{W}_2\ \ \mathbf{W}_3\ \cdots\ \mathbf{W}_{N-L+1}]\ ;$$

b) the matrices

$$\begin{bmatrix} \mathbf{v}_1^T \\ \mathbf{v}_2^T \cdot \Lambda^{-1} \\ \mathbf{v}_3^T \cdot \Lambda^{-2} \\ \vdots \\ \mathbf{v}_L^T \cdot \Lambda^{-(L-1)} \end{bmatrix}$$

as well as

$$[\mathbf{W}_1\ \Lambda^{-1}\cdot\mathbf{W}_2\ \Lambda^{-2}\cdot\mathbf{W}_3 \cdots \Lambda^{-(N-L)}\cdot\mathbf{W}_{N-L+1}]$$

are derived from these representations;

c) the matrix **C** of the complex amplitudes is then determined in accordance with the relation $\mathbf{C}^T=\mathbf{H}_D\cdot$–***diag***$\{x_1,x_2,...,x_p\}$, where $\mathbf{x}=(x_1,x_2,...,x_p)^T$ designates the mean value of the column vectors of $[\mathbf{w}_1\ \Lambda^{-1}\cdot\mathbf{w}_2\ \Lambda^{-2}\cdot\mathbf{w}_3 \cdots \Lambda^{-(N-L)}\cdot\mathbf{w}_{N-L+1}]$ and $\mathbf{H}_D$ designates the mean value of the block matrices in

$$\begin{bmatrix} \mathbf{v}_1^T \\ \mathbf{v}_2^T \cdot \Lambda^{-1} \\ \mathbf{v}_3^T \cdot \Lambda^{-2} \\ \vdots \\ \mathbf{v}_L^T \cdot \Lambda^{-(L-1)} \end{bmatrix} .$$

7. Method according to one of the preceding claims, in which the matrix **F** is determined with the aid of the method of least squares.

8. Method according to one of the preceding claims, in which the matrix **F** is determined with the aid of the method of total least squares.

9. Method according to one of Claims 1 or 2, in which all attenuation constants are zero and, in place of the matrix $\mathbf{Y}_E$, the matrix

$$\begin{pmatrix} \mathbf{y}^T(0) & \mathbf{y}^T(1) & \cdots & \mathbf{y}^T(N-L) & \mathbf{y}^H(0) & \mathbf{y}^H(1) & \cdots & \mathbf{y}^H(N-L) \\ \mathbf{y}^T(1) & \mathbf{y}^T(2) & \cdots & \mathbf{y}^T(N-L+1) & \mathbf{y}^H(1) & \mathbf{y}^H(2) & \cdots & \mathbf{y}^H(N-L+1) \\ \vdots & \vdots & & \vdots & \vdots & \vdots & & \vdots \\ \mathbf{y}^T(L-1) & \mathbf{y}^T(L) & \cdots & \mathbf{y}^T(N-1) & \mathbf{y}^H(L-1) & \mathbf{y}^H(L) & \cdots & \mathbf{y}^H(N-1) \end{pmatrix}$$

is used.

10. Method according to Claim 9, in which the matrix **F** is determined subject to the secondary condition that all eigenvalues are of the magnitude one.

11. Method according to one of the preceding claims, characterized in that said method is used for the detection of ventricular late potentials in multichannel medicotechnical measurement methods.

12. Use according to Claim 11, characterized in that the measured data are obtained by multichannel, chronological sampling of signals originating from the heart, in the terminal portion of the QRS complex and in the ST section.

13. Use according to Claim 12, characterized in that before carrying out the high-resolution spectral analysis constant components and linear trends in the measured data are compensated.

14. Use according to one of Claims 11 to 13 in conjunction with one of Claims 5 or 6, characterized in that a sensor array having a defined spatial arrangement of the individual sensors is used, and in that the spatial

position of a source of specified signal components is determined from the complex amplitudes or, in a manner equivalent thereto, from the real amplitudes and phases of the oscillations, on the basis of the known spatial arrangement of the individual sensors.

**15.** Use according to Claim 14, in which pathological regions in the heart muscle which are associated with the creation of late potentials are localized.

**16.** Use according to one of the preceding claims in conjunction with one of Claims 5 or 6 for the visualization of the harmonic components in the measured data, characterized in that a synthetic signal which is generated by in-phase superposition of the determined oscillations is represented on an output device.

**17.** Electronic data-processing system for carrying out a multichannel high-resolution spectral analysis by a method according to one of Claims 1 to 10.

## Revendications

**1.** Procédé d'analyse spectrale pour réaliser l'analyse spectrale à haute résolution et à plusieurs canaux, caractérisé par le fait que

a) on observe M suites temporelles $y_i(n)$ de données de mesure des canaux de capteurs, avec $1 \leqq i \leqq M$ et $M > 1$, aux instants $0 \leqq n \leqq N-1$ et on les assemble pour former une matrice $Y_E$ ayant la forme

$$Y_E = \begin{bmatrix} y^T(0) & y^T(1) & \dots & y^T(N-L) \\ y^T(1) & y^T(2) & \dots & y^T(N-L+1) \\ \cdot & \cdot & & \cdot \\ \cdot & \cdot & & \cdot \\ \cdot & \cdot & & \cdot \\ y^T(L-1) & y^T(L) & \dots & y^T(N-1) \end{bmatrix}$$

avec $y(n) = (y_1(n),\dots,y_M(n))^T$,

b) on exécute une décomposition en valeurs singulières $Y_E = U.\Sigma.V^H$ de la matrice $Y_E$ et on soumet les valeurs singulières à une opération avec valeur de seuil, ce qui a pour effet que toutes les valeurs singulières de $Y_E$, dont la valeur absolue est inférieure à la valeur de seuil, sont remplacées par des zéros, et l'ensemble des p valeurs singulières dominantes, c'est-à-dire non remplacées par zéro, de $Y_E$ sont contenues dans la matrice de bloc $\Sigma_1$ de la décomposition

$$Y_E = (U_1 \ U_2) \ . \begin{pmatrix} \Sigma_1 & 0 \\ 0 & 0 \end{pmatrix} . \begin{pmatrix} V_1{}^H \\ V_2{}^H \end{pmatrix}$$

c) on détermine les matrices $\Theta_1$ et $\Theta_2$, pour lesquelles on a

$$U_1 . \Sigma_1{}^{1/2} = \begin{pmatrix} \Theta_1 \\ \theta_2 \end{pmatrix} = \begin{pmatrix} \theta_1 \\ \Theta_2 \end{pmatrix}$$

$\theta_1$ et $\theta_2$ désignant respectivement la première et la dernière matrices de bloc,

d) on détermine une matrice F, qui est une solution optimale du système d'équations en général redondantes $\Theta_1.F = \Theta_2$, les parties imaginaires des logarithmes naturels des valeurs propres de F représentant les fréquences spectrales recherchées, tandis que les parties réelles des logarithmes naturels

des valeurs propres de F sont des constantes d'amortissement associées à ces fréquences.

2. Procédé d'analyse spectrale pour réaliser l'analyse spectrale à haute résolution et à plusieurs canaux, selon lequel on observe M suites temporelles $y_i(n)$ de données de mesure des canaux de capteurs, avec $1 \leqq i \leqq M$ et $M > 1$, aux instants $0 \leqq n \leqq N-1$, caractérisé par le fait qu'on détermine des fréquences spectrales et des constantes d'amortissement associés à ces fréquences sous la forme de parties imaginaires ou de parties réelles des logarithmes naturels des valeurs propres d'une matrice F, qui est définie en tant que solution optimale d'un système d'équations en général redondant $\Theta_1.F = \Theta_2$, les matrices $\Theta_1$ et $\Theta_2$ étant définies par la relation

$$U_1 . \Sigma_1^{1/2} = \begin{pmatrix} \Theta_1 \\ \theta_2 \end{pmatrix} = \begin{pmatrix} \theta_1 \\ \Theta_2 \end{pmatrix}$$

dans laquelle $\theta_1$ et $\theta_2$ désignent respectivement la première et la dernière matrices de bloc, et dans laquelle les matrices $\Sigma_1$ et $U_1$ sont définies par une décomposition approximative en valeurs singulières

$$Y_E = (U_1 \ U_2) . \begin{pmatrix} \Sigma_1 & 0 \\ 0 & 0 \end{pmatrix} . \begin{pmatrix} V_1^H \\ V_2^H \end{pmatrix}$$

d'une matrice

$$Y_E = \begin{bmatrix} y^T(0) & y^T(1) & \dots & y^T(N-L) \\ y^T(1) & y^T(2) & \dots & y^T(N-L+1) \\ . & . & & . \\ . & . & & . \\ . & . & & . \\ y^T(L-1) & y^T(L) & \dots & y^T(N-1) \end{bmatrix}$$

avec $y(n) = (y_1(n),...,y_M(n))^T$, et la décomposition approximative en valeurs singulières étant déterminée à partir d'une division initiale en valeurs singulières $y_E = U.\Sigma.V^H$ par le fait qu'on soumet les valeurs singulières à une opération à valeur de seuil, ce qui a pour effet que toutes les valeurs singulières de $Y_E$, dont la valeur absolue est inférieure à la valeur de seuil, sont remplacées par zéro, et l'ensemble des p valeurs singulières dominantes, c'est-à-dire non remplacées par zéro, de $Y_E$ sont contenues dans la matrice de bloc $\Sigma_1$ de la décomposition approximative.

3. Procédé d'analyse spectrale pour réaliser l'analyse spectrale à haute résolution et à plusieurs canaux, caractérisé par le fait que

a) on observe M suites temporelles $y_i(n)$ de données de mesure des canaux de capteurs, avec $1 \leqq i \leqq M$ et $M > 1$, aux instants $0 \leqq n \leqq N-1$ et on les rassemble pour former une matrice $Y_T$ ayant la forme

$$Y_T = \begin{bmatrix} y(0) & y(1) & \dots & y(N-L) \\ y(1) & y(2) & \dots & y(N-L+1) \\ \cdot & \cdot & & \cdot \\ \cdot & \cdot & & \cdot \\ \cdot & \cdot & & \cdot \\ y(L-1) & y(L) & \dots & y(N-1) \end{bmatrix}$$

avec $y(n) = (y_1(n),\dots,y_M(n))^T$,
b) on exécute une décomposition en valeurs singulières $y_T = U.\Sigma.V^H$ de la matrice $Y_T$ et on soumet les valeurs singulières à une opération avec valeur de seuil, ce qui a pour effet que toutes les valeurs singulières de $Y_T$, dont la valeur absolue est inférieure à la valeur de seuil, sont remplacées par des zéros, et l'ensemble des p valeurs singulières dominantes, c'est-à-dire non remplacées par zéro, de $Y_T$ sont contenues dans la matrice de bloc $\Sigma_1$ de la décomposition

$$Y_T = (U_1 \;\; U_2) \; . \; \begin{pmatrix} \Sigma_1 & 0 \\ 0 & 0 \end{pmatrix} . \begin{pmatrix} V_1^H \\ V_2^H \end{pmatrix}$$

c) on détermine celles des matrices $\Theta_1$ et $\Theta_2$, pour lesquelles on a

$$U_1.\Sigma_1^{1/2} = \begin{pmatrix} \Theta_1 \\ \theta_2 \end{pmatrix} = \begin{pmatrix} \theta_1 \\ \Theta_2 \end{pmatrix}$$

$\theta_1$ et $\theta_2$ désignant respectivement la première et la dernière matrices de bloc,
d) on détermine une matrice F, qui est une solution optimale du système d'équations en général redondantes $\Theta_1.F = \Theta_2$, les parties imaginaires des logarithmes naturels des valeurs propres de F représentant les fréquences spectrales recherchées, tandis que les parties réelles des logarithmes naturels des valeurs propres de F sont des constantes d'amortissement associées à ces fréquences.

**4.** Procédé d'analyse spectrale pour réaliser l'analyse spectrale à haute résolution et à plusieurs canaux, selon lequel on observe M suites temporelles $y_i(n)$ de données de mesure des canaux de capteurs, avec $1 \leqq i \leqq M$ et $M > 1$, aux instants $0 \leqq n \leqq N-1$, caractérisé par le fait qu'on détermine des fréquences spectrales et des constantes d'amortissement associées à ces fréquences sous la forme de parties imaginaires ou de parties réelles des logarithmes naturels des valeurs propres d'une matrice F, qui est définie en tant que solution optimale d'un système d'équations en général redondant $\Theta_1.F = \Theta_2$, les matrices $\Theta_1$ et $\Theta_2$ étant définies par la relation

$$U_1.\Sigma_1^{1/2} = \begin{pmatrix} \Theta_1 \\ \theta_2 \end{pmatrix} = \begin{pmatrix} \theta_1 \\ \Theta_2 \end{pmatrix}$$

dans laquelle $\theta_1$ et $\theta_2$ désignent respectivement la première et la dernière matrices de bloc, et dans laquelle les matrices $\Sigma_1$ et $U_1$ sont définies par une décomposition approximative en valeurs singulières

$$Y_T = (U_1 \; U_2) \cdot \begin{pmatrix} \Sigma_1 & 0 \\ 0 & 0 \end{pmatrix} \cdot \begin{pmatrix} V_1^H \\ V_2^H \end{pmatrix}$$

d'une matrice

$$Y_T = \begin{bmatrix} y(0) & y(1) & \dots & y(N-L) \\ y(1) & y(2) & \dots & y(N-L+1) \\ \cdot & \cdot & & \cdot \\ \cdot & \cdot & & \cdot \\ \cdot & \cdot & & \cdot \\ y(L-1) & y(L) & \dots & y(N-1) \end{bmatrix}$$

avec $y(n) = (y_1(n),...,y_M(n))^T$, et selon lequel la décomposition approximative en valeurs singulières est déterminée à partir d'une décomposition initiale en valeurs singulières $y_T = U.\Sigma.V^H$ par le fait qu'on soumet les valeurs singulières à une opération à valeur de seuil, ce qui a pour effet que toutes les valeurs singulières de $Y_T$, dont la valeur absolue est inférieure à la valeur de seuil, sont remplacées par zéro, et l'ensemble des p valeurs singulières dominantes, c'est-à-dire non remplacées par zéro, de $Y_T$ sont contenues dans la matrice de bloc $\Sigma_1$ de la decomposition approximative.

**5.** Procédé suivant l'une des revendications 1 ou 2, caractérisé par le fait que la matrice C des amplitudes de valeur complexe $c_{j,i}$ des oscillations associées aux fréquences spectrales j = 1,...,p dans le canal de capteur i = 1,...,M, sont déterminées par la mise en oeuvre des étapes suivantes :

a) à partir de la transformation de similitude $F = S.\Lambda.S^{-1}$ de la matrice F et de sa forme diagonale $\Lambda$, on crée les matrices

$$U_1.\Sigma_1^{1/2}.S \text{ et } S^{-1}.\Sigma_1^{1/2}.V_1^H$$

et on les divise en des matrices de bloc

$$U_1.\Sigma_1^{1/2}.S = \begin{bmatrix} v_1^T \\ v_2^T \\ v_3^T \\ \cdot \\ \cdot \\ \cdot \\ v_L^T \end{bmatrix}$$

et

$$S^{-1}.\Sigma_1^{1/2}.V_1^H = [W_1 \; W_2 \; W_3 ... W_{N-L+1}]$$

b) à partir de ces représentations on établit les matrices

$$\begin{bmatrix} v_1^T \\ v_2^T.\Lambda^{-1} \\ v_3^T.\Lambda^{-2} \\ . \\ . \\ . \\ v_L^T.\Lambda^{-(L-1)} \end{bmatrix}$$

et

$$[W_1\ \Lambda^{-1}.W_2\ \Lambda^{-2}.W_3 \ldots \Lambda^{-(N-L)}.W_{N-L+1}]$$

c) on détermine ensuite la matrice C des amplitudes complexes conformément à la relation C = diag$\{h_1,h_2...,h_p\}.X_D$, $h^T = (h_1,h_2...,h_p)$ représentant la valeur moyenne des vecteurs de lignes de

$$\begin{bmatrix} v_1^T \\ v_2^T.\Lambda^{-1} \\ v_3^T.\Lambda^{-2} \\ . \\ . \\ . \\ v_L^T.\Lambda^{-(L-1)} \end{bmatrix}$$

et $X_D$ désignant la valeur moyenne des matrices de bloc dans $[W_1\ \Lambda^{-1}.W_2\ \Lambda^{-2}.W_3 \ldots \Lambda^{-(N-L)}.W_{N-L+1}]$.

**6.** Procédé suivant l'une des revendications 3 ou 4, caractérisé par le fait que la matrice C des amplitudes de valeur complexe $c_{j,i}$ des oscillations associées aux fréquences spectrales j - 1,...,p dans le canal de capteur i = 1,...,M, sont déterminées par la mise en oeuvre des étapes suivantes :

a) à partir de la transformation de similitude $F = S.\Lambda.S^{-1}$ de la matrice F et de sa forme diagonale $\Lambda$, on crée les matrices

$$U_1.\Sigma_1^{1/2}.S \text{ et } S^{-1}.\Sigma_1^{1/2}.V_1^H$$

et on les divise en des matrices de bloc

$$U_1.\Sigma_1^{1/2}.S = \begin{bmatrix} V_1^T \\ V_2^T \\ V_3^T \\ . \\ . \\ . \\ V_L^T \end{bmatrix}$$

et

$$S^{-1}.\Sigma_1^{1/2}.V_1^H = [w_1\ w_2\ w_3 \ldots w_{N-L+1}]$$

b) à partir de ces représentations on établit les matrices

$$\begin{bmatrix} V_1^T \\ V_2^T.\Lambda^{-1} \\ V_3^T.\Lambda^{-2} \\ . \\ . \\ . \\ V_L^T.\Lambda^{-(L-1)} \end{bmatrix}$$

et

$$[w_1\ \Lambda^{-1}.w_2\ \Lambda^{-2}.w_3 \ldots\ \Lambda^{-(N-L)}.w_{N-L+1}]$$

c) on détermine alors la matrice C des amplitudes complexes conformément à la relation $C^T = H_D.\mathrm{diag}\{x_1,x_2,\ldots,x_p\}$, $x = (x_1,x_2,\ldots,x_p)^T = (h_1,h_2,\ldots,h_p)$ représentant la valeur moyenne des vecteurs de colonnes de $[w_1\ \Lambda^{-1}.w_2\ \Lambda^{-2}.w_3 \ldots\ \Lambda^{-(N-L)}.w_{N-L+1}]$, et $H_D$ représentant la valeur moyenne des matrices de bloc dans

$$\begin{bmatrix} V_1^T \\ V_2^T.\Lambda^{-1} \\ V_3^T.\Lambda^{-2} \\ . \\ . \\ . \\ V_L^T.\Lambda^{-(L-1)} \end{bmatrix}$$

7. Procédé suivant l'une des revendications précédentes, selon lequel on détermine la matrice F à l'aide de la méthode des moindres carrés d'erreurs.

8. Procédé suivant l'une des revendications précédentes, selon lequel on détermine la matrice F à l'aide de la méthode des moindres carrés d'erreurs totaux (total least squares).

9. Procédé suivant l'une des revendications 1 ou 2, selon lequel toutes les constantes d'amortissement sont des zéros et à la place de la matrice $Y_E$, on utilise la matrice

$$\begin{bmatrix} y^T(0) & y^T(1) & \ldots & y^T(N-L) & y^H(0) & y^H(1) & \ldots & y^H(N-L) \\ y^T(1) & y^T(2) & \ldots & y^T(N-L+1) & y^H(1) & y^H(2) & \ldots & y^H(N-L+1) \\ . & . & & . & . & . & & . \\ . & . & & . & . & . & & . \\ . & . & & . & . & . & & . \\ y^T(L-1) & y^T(L) & \ldots & y^T(N-1) & y^H(L-1) & y^H(L) & \ldots & y^H(N-1) \end{bmatrix}.$$

10. Procédé suivant la revendication 9, selon lequel on détermine la matrice F avec la condition secondaire selon laquelle toutes les valeurs propres possèdent la valeur absolue un.

11. Procédé suivant l'une des revendications précédentes, caractérisé par le fait qu'on l'utilise pour la détection de potentiels ventriculaires tardifs dans des procédés de mesure multicanaux de la technique médicale.

12. Utilisation suivant la revendication 11, caractérisée par le fait qu'on obtient les données de mesure par échantillonnage temporelle, selon des canaux multiples, de signaux provenant du coeur, dans la composante terminale du complexe QRS et dans la section ST.

13. Utilisation suivant la revendication 12, caractérisée par le fait qu'avant l'exécution de l'analyse spectrale à haute résolution, on compense des composantes continues et des tendances linéaires dans les données de mesure.

14. Utilisation suivant l'une des revendications 11 à 13 en liaison avec l'une des revendications 5 ou 6, caractérisée par le fait qu'on utilise un réseau de capteurs avec une disposition spatiale définie des différents capteurs et qu'à partir des amplitudes complexes ou, ce qui est équivalent, à partir des amplitudes et des phases réelles des oscillations, on détermine, dans la position de base de la disposition spatiale connue des différents capteurs, la position spatiale d'une source de composantes déterminées de signaux.

15. Utilisation suivant la revendication 14, selon laquelle on localise des régions pathologiques dans le muscle cardiaque, qui sont associées à l'apparition de potentiels tardifs.

16. Utilisation suivant l'une des revendications précédentes, en liaison avec l'une des revendications 5 ou 6 pour la visualisation des composantes harmoniques dans les données de mesure, caractérisée par le fait qu'un signal synthétique, qui est produit par une superposition correcte en phase des oscillations déterminées, est représenté sur un appareil de sortie.

17. Système électronique de traitement de données, destiné à la mise en oeuvre d'une analyse spectrale à haute résolution et à plusieurs canaux par un procédé suivant l'une des revendications 1 à 10.

FIG 1

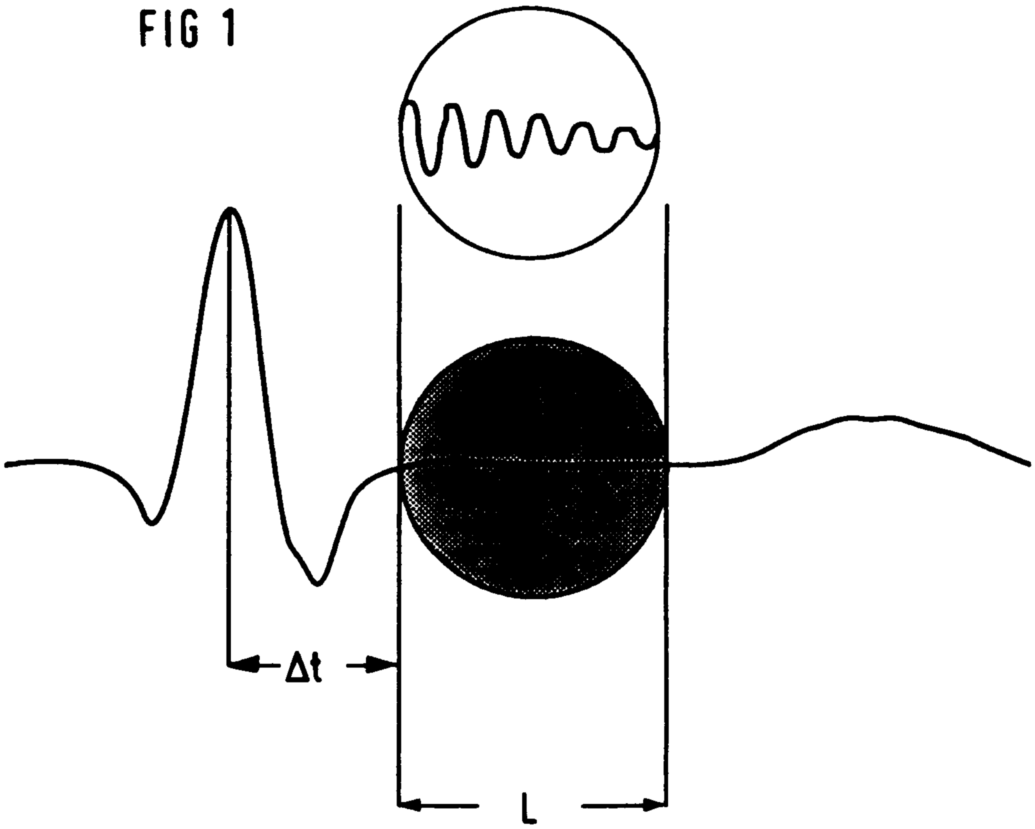


FIG 2

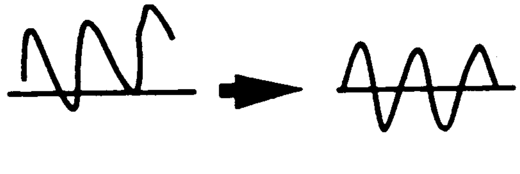

FIG 3

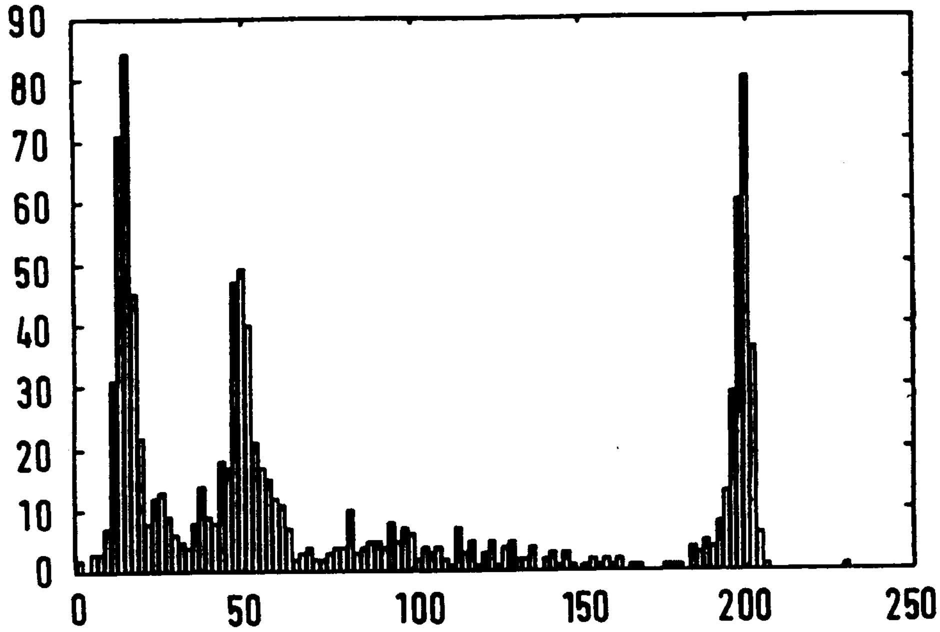
90
80
70
60
50
40
30
20
10
0
0
50
100
150
200
250

FIG 4

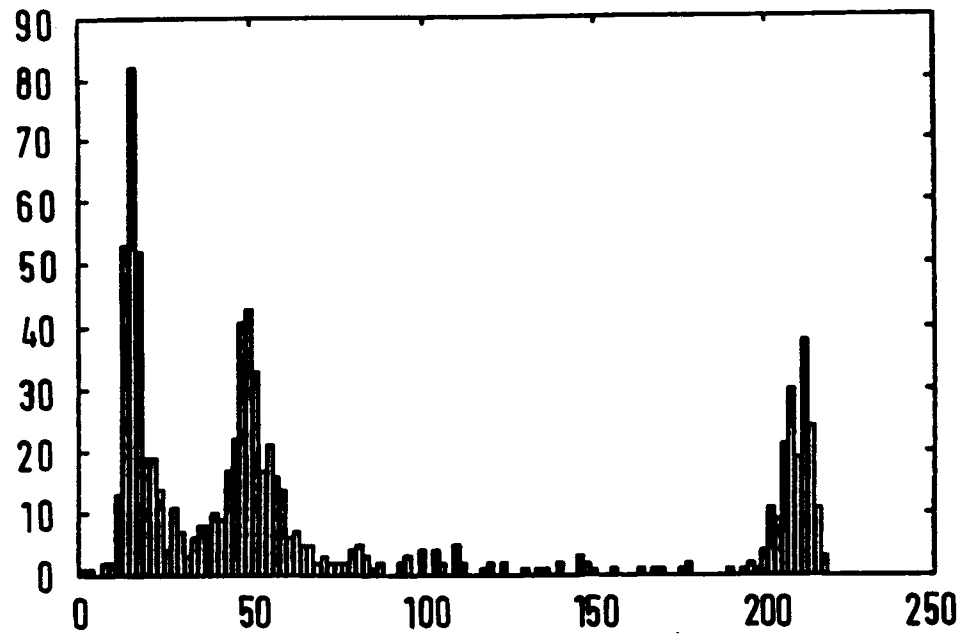
90
80
70
60
50
40
30
20
10
0
0
50
100
150
200
250

FIG 5

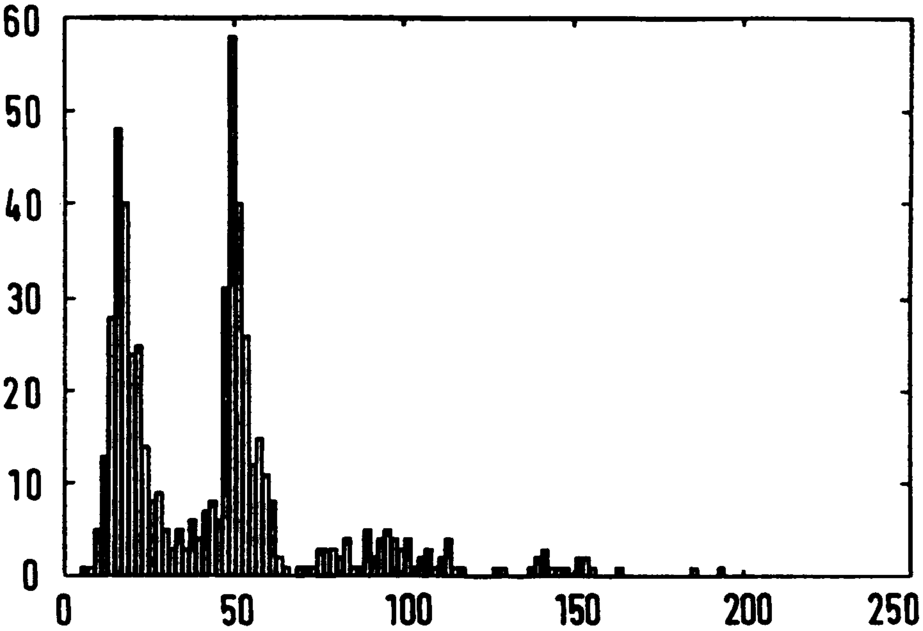
60
50
40
30
20
10
0
0
50
100
150
200
250

FIG 6

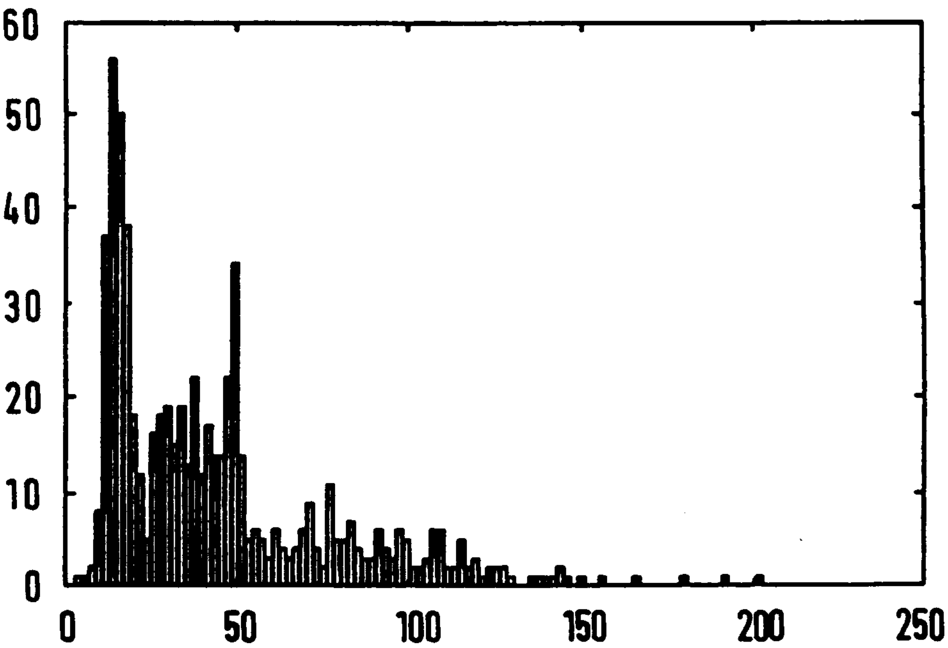
60
50
40
30
20
10
0
0
50
100
150
200
250